# EUROPEAN PATENT APPLICATION

(11) **EP 4 309 591 A1**
(43) Date of publication of application: **24.01.2024**
(21) Application number: 22770306.3
(22) Date of filing: 02.03.2022
(51) Int. Cl.: A61B 17/04

(54) **POSITIONER, CONVEYOR, TUNNEL-TYPE WIRE PASSING SYSTEM, AND OPERATING METHOD THEREFOR**

(30) Priority: 16.03.2021 CN 202110283061
(71) Applicant: SHANGHAI ARTHROPHIX MEDICAL TECHNOLOGY CO., LTD., Shanghai 201318 (CN)
(72) Inventor: QIN, Dan, Shanghai 201318 (CN); LI, Yonglong, Shanghai 201318 (CN); GE, Liang, Shanghai 201318 (CN); YOU, Mingzhe, Shanghai 201318 (CN)
(74) Representative: Patentship Patentanwaltsgesellschaft mbH
(86) International application number: PCT/CN2022/078814
(87) International publication number: WO 2022/193951

(57) **Abstract**

A positioner (1), a conveyor (2), a tunnel-type wire passing system and an operating method thereof. The positioner (1) includes a first locating rod (11), a second locating rod (12), a guide wire (13) and a drive unit (14). The first locating rod (11) defines a first channel (110) extending therethrough along its axial direction. The second locating rod (12) is connected to the first locating rod (11), and a distal end of the second locating rod (12) protrudes beyond a distal end of the first locating rod (11). The second locating rod (12) defines a second channel (120) extending therethrough along its axial direction. A distal end of the second channel (120) extending in a direction intersecting a direction in which a distal end of the first channel (110) extends. The guide wire (13) is movably inserted in the second channel (120) along the axial direction of the second locating rod (12). The guide wire (13) is provided at a distal end thereof with a connecting unit (131). The drive unit (14) is connected to the guide wire (13) and configured to drive the guide wire (13) to move in the axial direction of the second locating rod (12).

## Description

### TECHNICAL FIELD

The present invention relates to the field of medical devices and, in particular, to a positioner, a conveyor, a tunnel-type wire passing system, and an operating method thereof.

### BACKGROUND

Repair of torn soft tissue in the rotator cuff, knee, ankle or other body parts often requires not only suturing the soft tissue itself but also pressing the soft tissue against the bone (tying the soft tissue to the bone), for facilitated healing. At present, repair surgery of this type commonly utilizes suture anchors, which include a metal anchor for anchoring into a bone and a suture tied to the anchor for tying and pressing the soft tissue to the bone. Since the anchor is thicker and bulkier than the suture, such devices are not friendly to patients with small or osteoporotic bones. Improper anchor selection or inaccurate anchor placement would create risks of anchor dislodgement, inadequate traction, etc., which tend to cause secondary damage to the patient's affected part. Moreover, this therapy suffers from high surgical cost and complexity, and inadvertent damage after repair is extremely difficult to fix.

A safer surgical approach is to form a tunnel in a bone instead of using an anchor. A suture may be passed through the bone tunnel to accomplish soft tissue reattachment. However, currently, there are no suitable surgical tools for this purpose, and suture passage must rely on open surgery or arthroscopy-guided dural puncture, which involves very difficult surgical operations.

### SUMMARY OF THE INVENTION

It is an object of the present invention to overcome the problems associated with conventional anchor-based suture fixation by presenting a positioner, a conveyor, a tunnel-type wire passing system, and an operating method thereof.

To this end, the present invention provides a positioner comprising a first locating rod, a second locating rod, a guide wire and a drive unit,
the first locating rod defining a first channel extending therethrough along its axial direction,
the second locating rod coupled to the first locating rod, a distal end of the second locating rod protruding beyond a distal end of the first locating rod, the second locating rod defining a second channel extending therethrough along its axial direction, a distal end of the second channel extending in a direction intersecting a direction in which a distal end of the first channel extends;
the guide wire movably inserted in the second channel along the axial direction of the second locating rod, the guide wire provided at a distal end thereof with a connecting unit,
the drive unit coupled to the guide wire, the drive unit adapted to drive the guide wire to move in the axial direction of the second locating rod.

Optionally, the direction in which the distal end of the second channel extends may form an angle in the range of 10° to 180° with the direction in which the distal end of the first channel extends.

Optionally, the connecting unit may comprise a mechanical retainer or a bonding means.

Optionally, the mechanical retainer may be a hooking unit.

Optionally, the first locating rod may be coupled to the second locating rod so as to be movable in its own axial direction.

Optionally, the positioner may further comprise a locating rod sleeve defining a sleeve bore extending therethrough along its axial direction, an internal contour of the sleeve bore matching in shape an external contour of the first locating rod, wherein the first locating rod is movably inserted in the sleeve bore along the axial direction of the locating rod sleeve, and the second locating rod is fixedly attached to the locating rod sleeve.

Optionally, the second locating rod may comprise a curved section and a straight section joined to a proximal end of the curved section, the straight section extending in parallel to the axial direction of the first locating rod.

Optionally, the first locating rod may have an abutment surface at its distal end, the abutment surface adapted to be complementary in shape to an object of interest and for abutment with a surface of the object of interest.

Optionally, the first locating rod may be provided at its distal end with an engagement unit adapted to pierce the surface of the object of interest and thereby engage the object of interest.

Optionally, the positioner may further comprise a handle, which surrounds a proximal end of the first locating rod and extends in the form of a straight-line shape.

Optionally, the guide wire may be flexible, and the connecting unit may have rounded, smooth edges.

To the above end, the present invention also provides a conveyor comprising a conveyor shaft and a conveyor tip,
the conveyor tip disposed at a distal end of the conveyor shaft and adapted to accommodate a coil of a suture or an implant,
the conveyor shaft adapted to be movably inserted in the first channel in the positioner as defined above, the conveyor tip adapted to protrude out of the first locating rod from its distal end to allow the connecting unit of the positioner to catch the coil or the implant accommodated in the conveyor tip.

Optionally, the conveyor shaft may define a suture cavity extending therethrough along its axial direction, the suture cavity adapted to accommodate part of the suture.

Optionally, the conveyor may comprise a suture accommodating unit, which is coupled to a proximal end of the conveyor shaft and adapted to accommodate a proximal end portion of the suture.

Optionally, the conveyor may further comprise a stop unit secured to the conveyor shaft, the stop unit adapted to come into abutment with a corresponding portion of the positioner and thereby restrict distal displacement of the conveyor shaft relative to the first channel, wherein upon the stop unit coming into abutment with the corresponding portion of the positioner, the conveyor tip is located at the intersection of the direction in which the distal end of the second channel extends and the direction in which the distal end of the first channel extends.

Optionally, the conveyor tip may comprise a first recess for accommodating the coil and a second recess adapted for passage of the connecting unit therethrough.

Optionally, the conveyor tip may further comprise a retaining fork with a distal end defining a locating retainer and at least two fork prongs which are connected by the locating retainer to form a closed retaining slot for capturing the coil of the suture or accommodating the implant.

Optionally, the conveyor tip may comprise two of the retaining forks which are disposed in opposition to each other.

To the above end, the present invention also provides a tunnel-type wire passing system, which comprises the positioner as defined above and the conveyor as defined above, wherein the conveyor shaft in the conveyor is adapted to be inserted in the first channel of the positioner, and the connecting unit in the positioner is adapted to catch the coil or the implant accommodated in the conveyor tip.

To the above end, the present invention also provides a method of operating the system as defined above. The method comprises:
forming a second tunnel in an object of interest;
inserting the distal end of the second locating rod of the positioner into the second tunnel so that the direction in which the distal end of the second channel extends coincides with an axial direction of the second tunnel;
forming a first tunnel in the object of interest along a direction in which the first channel in the positioner extends so that the first tunnel intersects the second tunnel;
inserting the conveyor shaft of the conveyor into the first channel so that the distal end of the conveyor shaft is located within the first tunnel and that the conveyor tip of the conveyor is located at the intersection of the second tunnel and the first tunnel;
using the drive unit to drive the connecting unit to proximally move and catch the coil or the implant accommodated in the conveyor tip;
proximally pulling and withdrawing the conveyor, with the coil or the implant remaining in the second tunnel; and
proximally pulling the positioner until part of the coil or the implant is pulled out of the second tunnel.

Optionally, after the coil is pulled out of the second tunnel, the method may further comprise:
using the drive unit to drive the connecting unit to distally move; and
detaching the coil or the implant from the connecting unit, thereby separating it from the positioner.

In summary, the present invention provides a positioner, a conveyor, a tunnel-type wire passing system and a operating method thereof. The positioner includes a first locating rod, second locating rod, a guide wire and a drive unit. The first locating rod defines a first channel extending therethrough along its axial direction. The second locating rod is coupled to the first locating rod. A distal end of the second locating rod protrudes beyond a distal end of the first locating rod. The second locating rod defines a second channel extending therethrough along its axial direction. A distal end of the second channel extends in a direction intersecting a direction in which a distal end of the first channel extends. The guide wire is movably inserted in the second channel along the axial direction of the second locating rod. The guide wire provided is at a distal end thereof with a connecting unit. The drive unit is coupled to the guide wire and adapted to drive the guide wire to move in the axial direction of the second locating rod.

With this configuration, after the second tunnel is first formed in the object of interest, the distal end of the second locating rod may be inserted into the second tunnel, and a first tunnel first may be then formed in the object of interest along the direction of the first channel. As the direction of extension of the distal end of the second channel in the positioner intersects the direction of extension of the distal end of the first channel, the first tunnel will surely intersect the second tunnel achieving high locating accuracy that would be impossible for a percutaneous locating approach, without failure in hooking the suture that may occur when the first tunnel does not intersect the second tunnel. Additionally, the connecting unit provided at the distal end of the guide wire may catch the suture inserted into the first tunnel and pull the suture out from the second tunnel. In this way, the suture can be passed through the tunnels in the object of interest in a robust and reliable manner, without anchor dislodgement and other risks. The use of the positioner is simple, inexpensive and safe. Furthermore, since the first tunnel is formed at a later time than the second tunnel, the tool for forming the first tunnel may be used to clean debris generated during the formation of the second tunnel. This can prevent occlusion of the tunnel by the debris, which may impede the hooking and retaining of the suture.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1 is a schematic illustration of a tunnel-type wire passing system according to a first embodiment of the present invention.
Fig. 2 is a schematic axial cross-sectional view of the tunnel-type wire passing system according to the first embodiment of the present invention.
Fig. 3 is a schematic partial view of the tunnel-type wire passing system according to the first embodiment of the present invention.
Fig. 4 is an axial cross-sectional view of a positioner according to the first embodiment of the present invention.
Fig. 5 is a schematic illustration of a locating rod sleeve according to the first embodiment of the present invention.
Fig. 6 is a schematic illustration of a hooking unit according to the first embodiment of the present invention.
Fig. 7 is a schematic illustration of a conveyor according to the first embodiment of the present invention.
Fig. 8 is an enlarged partial view of a distal end of the conveyor of Fig. 7.
Fig. 9 schematically illustrates an application scenario of the tunnel-type wire passing system according to the first embodiment of the present invention.
Fig. 10 is a schematic illustration of a locking unit according to the first embodiment of the present invention.
Fig. 11 is a schematic illustration of a conveyor tip according to another preferred example of the first embodiment of the present invention.
Fig. 12 is a schematic illustration of a vertical locating rod according to a second embodiment of the present invention.
Fig. 13 is a schematic illustration of a first rotating wheel according to the second embodiment of the present invention.
Fig. 14 is a schematic illustration of a second rotating wheel and a toothed rack according to the second embodiment of the present invention.
Fig. 15 is a schematic exploded view of a conveyor according to a third embodiment of the present invention.
Fig. 16 is a schematic illustration of an outer suture bobbin group according to the third embodiment of the present invention.
Fig. 17 is a schematic illustration of an indication unit according to the third embodiment of the present invention.
Fig. 18 is a schematic illustration of a torsion spring according to the third embodiment of the present invention.
Fig. 19 is a schematic illustration of a conveyor according to a fourth embodiment of the present invention.
Fig. 20 is an enlarged partial view of the conveyor of Fig. 19.
Fig. 21 is a schematic partial view of a conveyor according to a fifth embodiment of the present invention.
Fig. 22 is a schematic overview of a suture accommodating unit according to the fifth embodiment of the present invention.
Fig. 23 schematically illustrates the internal structure of the suture accommodating unit according to the fifth embodiment of the present invention.
Fig. 24 is a schematic illustration of a double-leaf hook according to the sixth embodiment of the present invention.
Fig. 25 is a schematic illustration of the double-leaf hook with a suture anchor caught thereon according to the sixth embodiment of the present invention.
Fig. 26 is a schematic illustration of the double-leaf hook with a titanium suture button caught thereon according to the sixth embodiment of the present invention.
Fig. 27 is a schematic illustration of the double-leaf hook with a suture hooked and retained thereon according to the sixth embodiment of the present invention.
Fig. 28 is a schematic illustration of a flat spiral hook according to the sixth embodiment of the present invention.
Fig. 29 is a schematic illustration of the flat spiral hook with an interference screw retained thereon according to the sixth embodiment of the present invention.
Fig. 30 is a schematic illustration of a loop hook according to the sixth embodiment of the present invention.

In these figures,
1 denotes a positioner; 11, a horizontal locating rod; 110, a horizontal channel; 111, an abutment surface; 112, an engagement unit; 12, a vertical locating rod; 120, a vertical channel; 121, a curved section; 122, a straight section; 13, a guide wire; 131, a hooking unit; 132, a connecting link; 133, a double-leaf hook; 1331, an extension; 1332, an adapter portion; 134, a flat spiral hook; 135, a loop hook; 14, a drive unit; 141, a push button; 142, a first rotating wheel; 15, a locating rod sleeve; 150, a sleeve bore; 151, a vertical locating rod accommodating bore; 152, a locating recess; 153, a vertical channel extension; 16, a handle; 161, a locating projection; 162, a rotating shaft accommodating ring; 17, a locking unit; 171, a turning wheel; 1710, a turning wheel body; 1711, a rotating shaft; 1712, a stopper; 172, a push member; 181, a second rotating wheel; 182, a toothed rack;
2, a conveyor; 21, a conveyor shaft; 210, a suture cavity; 22, a conveyor tip; 221, a first recess; 222, a second recess; 223, a sloped guide surface; 23, a suture accommodating unit; 231, a suture bobbin; 2311, a push post; 2312, an outer bobbin; 2313, an inner bobbin; 2314, teeth; 2315, an outer suture bobbin group; 2316, an inner suture bobbin group; 2317, a stop plate; 232, a case; 2320, a window; 233, a suture spacer; 234, a first suture slot; 235, a second suture slot; 236, an observation area; 237, a suture bobbin axle; 238, a suture hole; 2370, a circumferential opening; 2371, a rotation stop unit; 24, a stop unit; 250, a retaining slot; 251, a locating retainer; 252, a fork prong; 26, an indication unit; 261, a torsion spring; 262, an indication mark; 27, a sleeve; 270, a push rod passage; 271, an opening; 28, a push rod; 281, an expanded section; 29, a retraction drive unit;
31, a coil; 32, a suture; 33, a suture tie; 34, a suture anchor; 341, a tensioning suture; 35, a titanium suture button; 351, a button body; 352, a tensioning suture; 36, an interference screw;
4, an object of interest; 41, a vertical tunnel; and 42, a horizontal tunnel.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, structures shown in the figures are usually part of actual structures. In particular, as the figures tend to have distinct emphases, they are often drawn to different scales.

As used herein, the singular forms "a", "an" and "the" include plural referents. As used herein, the term "or" is generally employed in the sense of "and/or", "several" of "at least one" and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal" generally refer to an end closer to an operator, and the term "distal" generally refer to an end closer to a lesion in a patient. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

The present invention seeks to overcome the problems associated with conventional anchor-based suture fixation by presenting a positioner, a conveyor, a tunnel-type wire passing system, and an operating method thereof.

According to embodiments of the present invention, the tunnel-type wire passing system is principally used to form a bone tunnel in a bone and passing a suture or implant (e.g., an interference screw, a titanium suture button, etc.) through the bone tunnel. Of course, the tunnel-type wire passing system is not limited to being used to pass a suture through a tunnel in a bone and may be used to pass a suture through a tunnel in another object.

The following description is set forth with reference to the accompanying drawings in the context of forming a bone tunnel in a bone, as an example. For ease of description, in the following, horizontal locating rods are also referred to as first locating rods, and vertical locating rods as second locating rods, horizontal tunnels as first tunnels and vertical tunnels as second tunnels, as an example. Based on the above concept, those skilled in the art may also refer to the horizontal components as second components and the vertical components as first components, without departing from the scope of the present invention.

### Embodiment 1

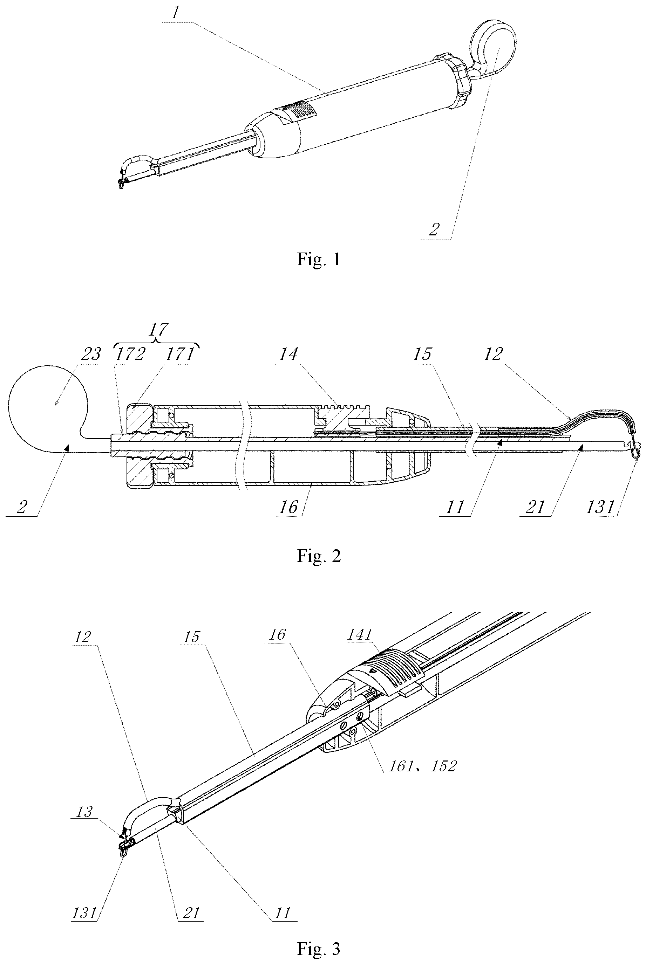

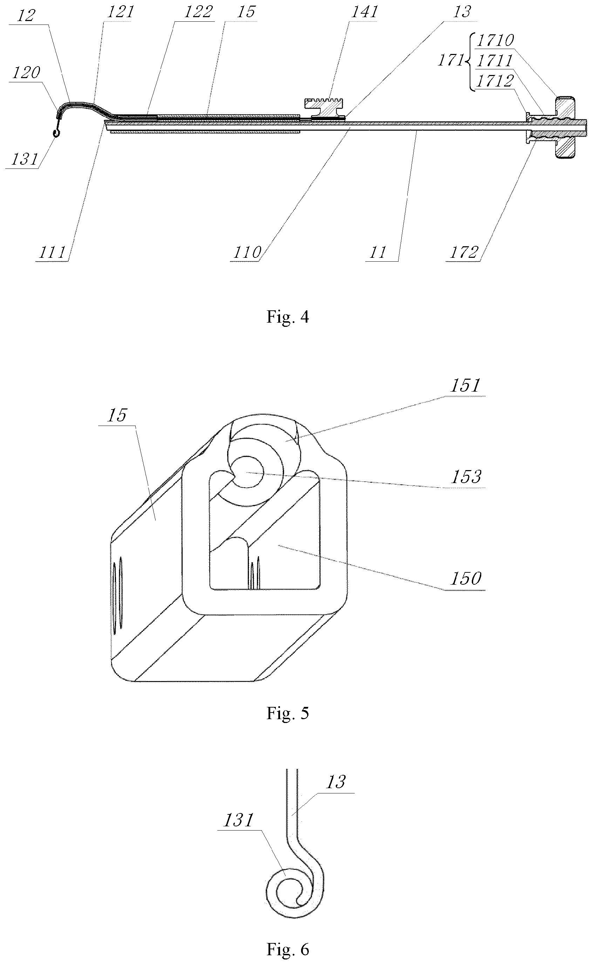

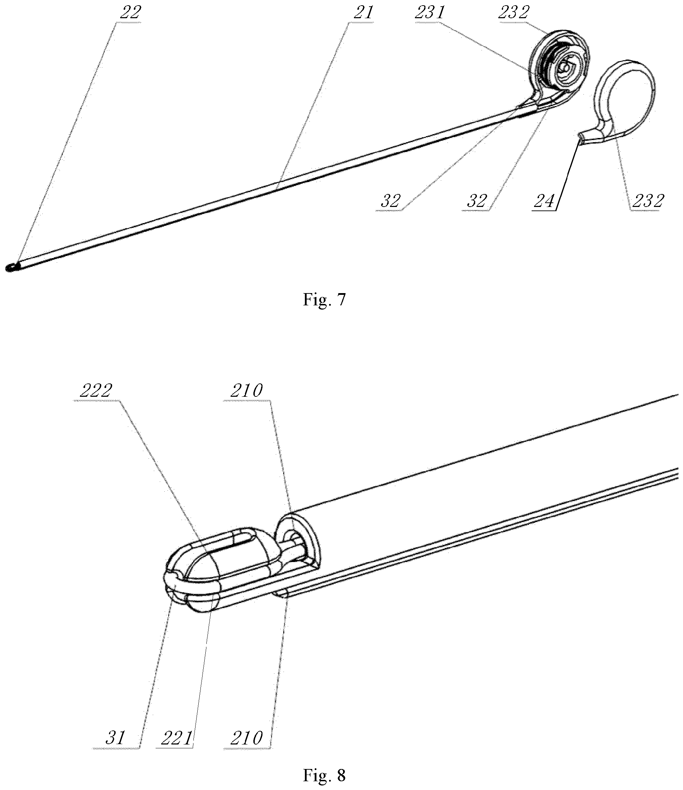

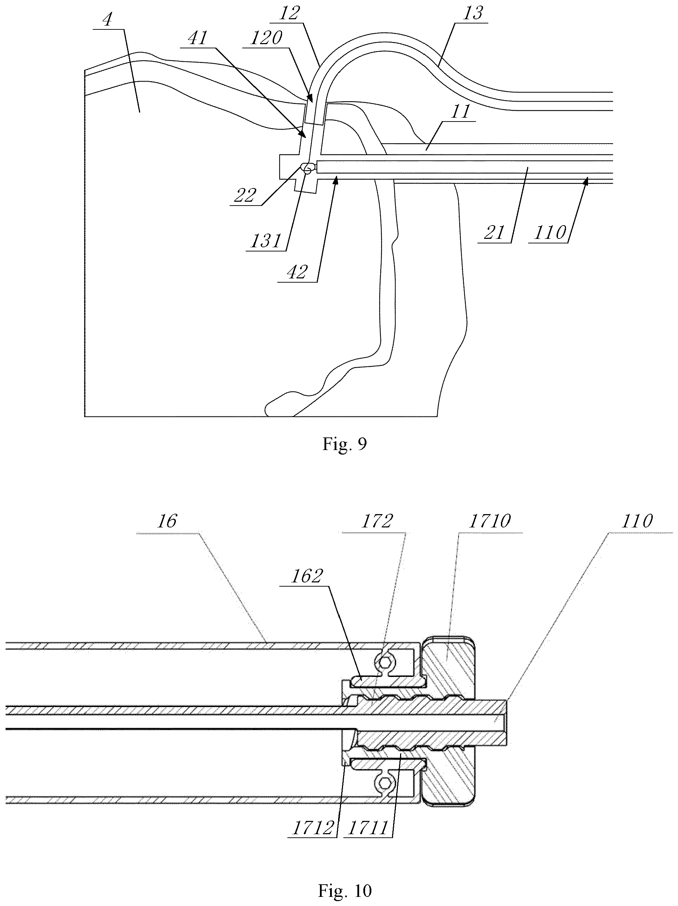

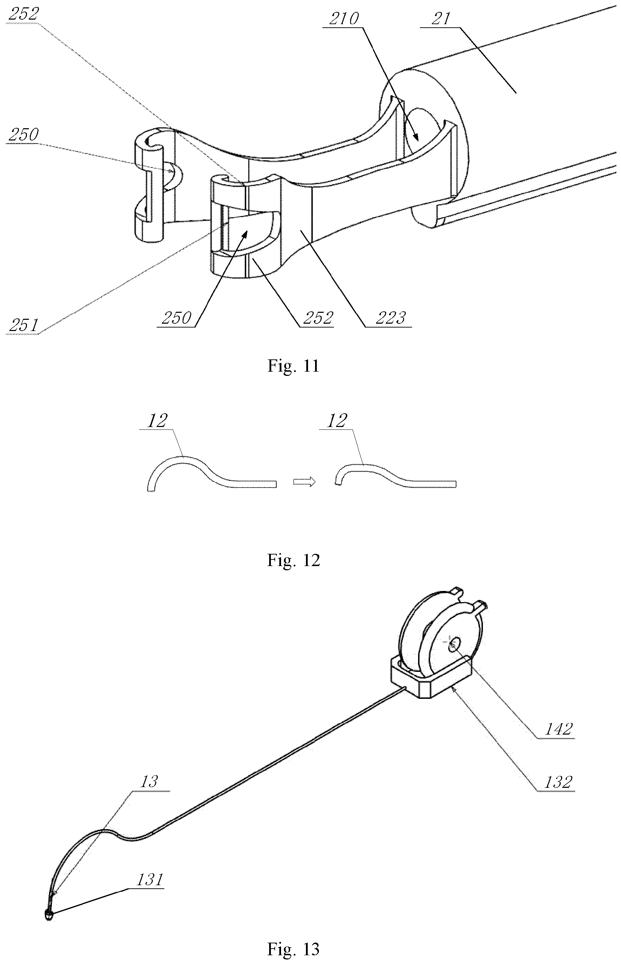

Reference is now made to Figs. 1 to 11. Fig. 1 is a schematic illustration of a tunnel-type wire passing system according to a first embodiment of the present invention. Fig. 2 is a schematic axial cross-sectional view of the tunnel-type wire passing system according to the first embodiment of the present invention. Fig. 3 is a schematic partial view of the tunnel-type wire passing system according to the first embodiment of the present invention. Fig. 4 is an axial cross-sectional view of a positioner according to the first embodiment of the present invention. Fig. 5 is a schematic illustration of a locating rod sleeve according to the first embodiment of the present invention. Fig. 6 is a schematic illustration of a hooking unit according to the first embodiment of the present invention. Fig. 7 is a schematic illustration of a conveyor according to the first embodiment of the present invention. Fig. 8 is an enlarged partial view of a distal end of the conveyor of Fig. 7. Fig. 9 schematically illustrates an application scenario of the tunnel-type wire passing system according to the first embodiment of the present invention. Fig. 10 is a schematic illustration of a locking unit according to the first embodiment of the present invention. Fig. 11 is a schematic illustration of a conveyor tip according to another preferred example of the first embodiment of the present invention.

As shown in Figs. 1 to 3, in this first embodiment of the present invention, there is provided a tunnel-type wire passing system, which includes a positioner 1 and a conveyor 2. The conveyor 2 is detachable from the positioner 1.

Referring to Fig. 4, in conjunction with Figs. 2 and 3, the positioner 1 includes a horizontal locating rod 11, a vertical locating rod 12 and a connecting member. The horizontal locating rod 11 defines a horizontal channel 110 extending therethrough along its axial direction. The vertical locating rod 12 is coupled to the horizontal locating rod 11, and a distal end of the vertical locating rod 12 protrudes beyond a distal end of the horizontal locating rod 11. The vertical locating rod 12 defines a vertical channel 120 extending therethrough along its axial direction. The direction of extension of the distal end of the vertical channel 120 intersects the direction of extension of the distal end of the horizontal channel 110. The connecting member is movably inserted in the vertical channel 120 of the vertical locating rod 12 along the axial direction thereof and is adapted to connect a suture or implant. The positioner 1 further includes a drive unit 14, which is coupled to a proximal end of the connecting member and adapted to drive the connecting member to move along the axial direction of the vertical locating rod 120. In an exemplary implementation, the connecting member is a guide wire 13 defining a connecting unit at its distal end, which is adapted for connection with the suture or implant. Optionally, the connecting unit may include a mechanical retainer or a bonding means. The mechanical retainer is adapted to mechanically hook and retain the suture or implant, and the bonding means is adapted to adhesively bonding the suture or implant. Preferably, the mechanical retainer is a hooking unit 131 capable of hooking and retaining the suture or implant.

It is to be noted that the axial directions of the horizontal and vertical locating rods 11, 12 are not limited to be straight-line directions, and if the horizontal or vertical locating rod 11, 12 is curved, then its axis will be a curved line and its axial direction will be a curved direction. Accordingly, the horizontal and vertical channels 110, 120 are also not limited to being straight line-shaped. Moreover, the directions of extension of the distal ends of the horizontal and vertical channels 110, 120 refer to the directions of extension of their axes at the distal ends. If the horizontal and vertical channels 110, 120 extend at their distal ends along straight lines defined by their axes, then the directions of extension of the distal ends refer to the directions of these straight lines. If the horizontal and vertical channels 110, 120 extend at their distal ends along curved lines defined by their axes, then the directions of extension of the distal ends refer to the directions of straight lines tangent to the axes at the distal endpoints. Here, by "the direction of extension of the distal end of the vertical channel 120 intersects the direction of extension of the distal end of the horizontal channel 110", it is intended to mean that the directions of extension of the distal ends of the horizontal and vertical channels 110, 120 pass through common point(s). The intersection may include geometric intersection and extension along a single line, but does not include parallelism and noncoplanarity.

Referring to Figs. 7 and 8, the conveyor 2 includes a conveyor shaft 21 and a conveyor tip 22. The conveyor tip 22 is disposed at a distal end of the conveyor shaft 21 and adapted to accommodate a coil 31 formed by the suture 32 or the implant. The conveyor shaft 21 is adapted to be movably inserted in the horizontal channel 110 of the positioner 1, and the conveyor tip 22 is adapted to protrude out of the horizontal locating rod 11 from its distal end so that the hooking unit 131 of the positioner 1 can hook and retain the coil 31 or the implant accommodated in the conveyor tip 22. In an exemplary implementation, the conveyor shaft 21 defines a suture cavity 210 extending along its axial direction and adapted to accommodate part of the suture 32. Preferably, the conveyor tip 22 defines a first recess 221 for accommodating and retaining the coil 31 and a second recess 222 for passage of the connecting unit (e.g., the hooking unit 131, etc.) therethrough. The present invention is not limited to any particular shape of the conveyor tip 22. Preferably, the conveyor tip 22 has a rounded, blunt distal end, which will not cause damage to a bone tunnel when rubbing against it. The coil 31 is formed by a distal section of the suture 32, which is wound on the conveyor tip 22. The suture cavity 210 extends along the axial direction of the conveyor shaft 21. In some implementations, the suture cavity 210 is a tubular lumen extending through the conveyor shaft 21. Of course, in some alternative implementations, the suture cavity 210 may be a radially open channel formed on the conveyor shaft 21. The present invention is not limited to any particular configuration of the suture cavity 210.

The conveyor shaft 21 of the conveyor 2 is adapted for insertion into the horizontal channel 110 in the positioner 1, and the connecting unit of the positioner 1 is adapted for catching the coil 31 accommodated in the conveyor tip 22 of the conveyor 2. Referring to Fig. 9, the distal end of the vertical locating rod 12 may be inserted into a vertical tunnel 41 formed in advance in an object 4 of interest (e.g., a humerus) to form a horizontal tunnel 42 in the object 4 of interest along the direction of the horizontal channel 110. As the direction of extension of the distal end of the vertical channel 120 in the positioner 1 intersects the direction of extension of the distal end of the horizontal channel 110, the horizontal tunnel 42 will surely intersect the vertical tunnel 41, achieving high locating accuracy that would be impossible for a percutaneous locating approach, without failure in hooking the suture that may occur when the horizontal tunnel 42 does not intersect the vertical tunnel 41. Further, the connecting unit may catch the coil 31 inserted into the horizontal tunnel 42 and pull the suture 32 out from the vertical tunnel 41. In this way, the suture 32 can be passed through the tunnels in the object 4 of interest in a robust and reliable manner, without anchor dislodgement and other risks. The use of the positioner 1 is simple, inexpensive and safe. Furthermore, since the horizontal tunnel 42 is formed at a later time than the vertical tunnel 41, the tool for forming the horizontal tunnel 42 may be used to clean debris generated during the formation of the vertical tunnel 41. This can prevent occlusion of the tunnel by the debris, which may impede the hooking and retaining of the suture 32.

Referring to Fig. 6, optionally, the guide wire 13 may be flexible, and the connecting unit may have rounded, smooth edges. The following description is set forth in the context of the connecting unit being implemented as the hooking unit 131, as an example. Since the hooking unit 131 can directly hook and retain the coil 31 of the suture 32, and because the suture 32 is typically soft, edges of the hooking unit 131 must be rounded and smoothed to avoid cutting the suture 32. The flexible guide wire 13 can bend in conformity with the geometry of the vertical channel 120. This enables the positioner 1 to have a reduced overall radial dimension at the distal end, which can enhance passability and allow smaller incisions to be made. Moreover, the flexibility of the guide wire 13 imparts to it enhanced traction capabilities. For example, the guide wire 13 may withstand a tensile/compressive stress greater than 450 MPa. The guide wire 13 may be made of, for example, a metal for medical use, such as 30Crl3, 40Crl3, 12Cr18Ni9, 12Cr18Ni10 or 12Cr17Ni7. In addition, in order to enable smooth movement of the hooking unit 131 in the vertical tunnel 41, its hardness is preferably not below 45 HRC. For example, it may be formed of a stainless steel wire of 0.1-2 mm (preferably, 0.5-0.6 mm), which may be bent into a predetermined shape and then hardened by thermal treatment. Of course, the hooking unit 131 having hardness not below 45 HRC can also be used to hook and retain a hard guide wire or implant, such as a nickel-titanium alloy wire or a titanium suture button. Preferably, the hooking unit 131 may be made of the same material as the guide wire 13, but the portion of the guide wire 13 may not have been subjected to thermal treatment and thus maintain its original toughness. It is to be noted that, while the hooking unit 131 is shown in Fig. 6 in a side view, it actually has a height measured in the direction perpendicular to the paper plane. That is, the hooking unit 131 appears substantially like a spring, and the coil 31 can be easily wound through the hollow of the hooking unit 131. It will be appreciated that the above-discussed configurations of the guide wire 13 and the hooking unit 131 are merely preferred exemplary implementations, and the guide wire 13 and the hooking unit 131 are not limited to those configurations in any sense. Those skilled in the art can choose other conventional configurations of the guide wire 13 and the hooking unit 131, and the present invention is not limited to any such configurations.

Further, the horizontal locating rod 11 is coupled to the vertical locating rod 12 while being able to move along its own axial direction so that after the vertical locating rod 12 is inserted into the vertical tunnel 41, the horizontal locating rod 11 can move along its own axial direction into abutment with the object 4 of interest. Referring to Fig. 5, in conjunction with Figs. 3 and 4, in an exemplary implementation, the positioner 1 further includes a locating rod sleeve 15 defining a sleeve bore 150 extending therethrough along its axial direction. An internal contour of the sleeve bore 150 matches in shape an external contour of the horizontal locating rod 11, and the horizontal locating rod 11 is movably inserted in the sleeve bore 150 along the axial direction of the locating rod sleeve 15. The vertical locating rod 12 is fixedly attached to the locating rod sleeve 15. The locating rod sleeve 15 can restrict radial freedom of movement of the horizontal locating rod 11, preventing its uncontrolled wobbling.

Optionally, the vertical locating rod 12 may include a curved section 121 and a straight section 122 joined to a proximal end of the curved section 121. The straight section 122 is parallel to the axial direction of the horizontal locating rod 11. For ease of use, the positioner 1 is desired to extend overall along a straight line, in order not to interfere with other parts of the patient's body. Accordingly, the straight section 122 of the vertical locating rod 12 is configured to be parallel to the axial direction of the horizontal locating rod 11. Preferably, the locating rod sleeve 15 further includes a vertical locating rod accommodating bore 151 extending along its axial direction. An internal contour of the vertical locating rod accommodating bore 151 matches in shape an external contour of the vertical locating rod 12. The vertical locating rod accommodating bore 151 is adapted to accommodate a proximal portion of the vertical locating rod 12. The straight section 122 may be inserted into the vertical locating rod accommodating bore 151 and then secured thereto (e.g., by welding or crimping) once the direction of extension of the distal end of the vertical channel 120 is adjusted to intersect the direction of extension of the distal end of the horizontal channel 110 and the intersection has been confirmed. Of course, this may be accomplished either during the fabrication of the positioner 1, or immediately before the positioner 1 is to be used. The present invention is not limited in this regard. It is to be noted that the curved section 121 may be adapted in shape to various objects 4 of interest. Preferably, the curved section 121 has a short distal straight portion, which can be easily inserted into the vertical tunnel 41 in the object 4 of interest while allowing the vertical tunnel 41 to have a smaller diameter.

Optionally, for ease of use, the positioner 1 is desired to have a relatively large length. However, for ease of assembly, the straight section 122 of the vertical locating rod 12 is desired not to be excessively long. To this end, the locating rod sleeve 15 may further define a vertical channel extension 153, which is configured in coaxiality with the vertical locating rod accommodating bore 151 and located at a proximal end thereof. The straight section 122 may be inserted into the vertical locating rod accommodating bore 151 so that the direction of extension of a proximal end of the vertical channel 120 coincides with an axial direction of the vertical channel extension 153, allowing proximal insertion of the guide wire 13 from the vertical channel extension 153.

Preferably, the direction of extension of the distal end of the vertical channel 120 intersects the direction of extension of the distal end of the horizontal channel 110 at an angle in the range of 10° to 180°. Angles in this range are suitable for more applications.

Referring to Figs. 1 and 2, preferably, the positioner 1 further includes a handle 16, which surrounds a proximal end of the horizontal locating rod 11 and extends in the form of a straight line shape. The handle 16 may be fixedly attached to the locating rod sleeve 15. In an exemplary implementation, the handle 16 includes two detachable halves, which can be assembled together by means of mutually locatable pairs of snap features. Preferably, a locating projection 161 is provided on the handle 16, and a mating locating recess 152 is provided on the locating rod sleeve 15. The handle 16 can be fixedly attached to the locating rod sleeve 15 by inserting the locating projection 161 into the locating recess 152. Additionally, a distal end of the handle 16 may match an outer circumferential contour of the locating rod sleeve 15. With this configuration, the locating rod sleeve 15 can be firmly secured within the handle 16. Of course, in some other examples, the handle 16 may be integrated with the locating rod sleeve 15, or an external contour of the locating rod sleeve 15 may define the handle 16, without departing from the scope of the present invention.

With continued reference to Figs. 1 and 2, in an alternative implementation, for example, the drive unit 14 may include, a push button 141, and the handle 16 may define a push button slot in which the push button 141 can be slid. The push button 141 is complementary in shape to push button slot. The push button slot extends along the axial direction of the horizontal locating rod 11. Once assembled in the push button slot, the push button 141 will be restricted thereby so that it can move only in the axial direction of the locating rod 11. In this way, radial wobbling can be avoided. The push button 141 may be secured to a proximal end of the guide wire 13, allowing an operator to driving the guide wire 13 to move forth and back in the axial direction of the vertical channel 120 by manipulating the push button 141.

In an optional implementation, the positioner 1 may further include a locking unit 17 adapted to drive the horizontal locating rod 11 to move distally to clamp the object 4 of interest together with the vertical locating rod 12. Referring to Fig. 9, it will be appreciated that after the vertical locating rod 12 is inserted into the vertical tunnel 41 in the object 4 of interest, bringing the horizontal locating rod 11 into distal abutment with the object 4 of interest by distally moving the horizontal locating rod 11 relative to the vertical locating rod 12 can enable the horizontal locating rod 11 and the vertical locating rod 12 to together clamp the object 4 of interest. At this time, the operator is allowed to take his/her hands off the positioner 1 rather than having to always hold it by hand.

Referring to Fig. 10, in an exemplary implementation, the locking unit 17 includes a turning wheel 171 and a push member 172. The turning wheel 171 has an internal thread, and the push member 172 has a matching outer thread engaging the internal thread of the turning wheel 171. The push member 172 is secured to the horizontal locating rod 11, and the turning wheel 171 is rotatably coupled to the locating rod sleeve 15 and restricted so as to displace in the axial direction of the locating rod sleeve 15. Since the vertical locating rod 12 and the locating rod sleeve 15 are maintained stationary relative to each other once they are assembled together, rotating the turning wheel 171 can drive the horizontal locating rod 11 to forth and back along its own axial direction.

With this configuration, before the horizontal tunnel 42 is formed in the object of interest along the direction of the horizontal channel, the positioner 1 may be secured to the object 4 of interest with the locking unit 17, allowing the operator to take his/her hands off the positioner 1 without having to always hold it by hand. In this way, on the one hand, the horizontal locating rod 11 can be brought into firm abutment with the object 4 of interest, allowing the direction of extension of the horizontal channel 110 to precisely intersect that of the vertical tunnel 41. This can facilitate the subsequent formation of the horizontal tunnel 42 along the horizontal channel 110 with a bone drill or another tool and thus achieve accurate locating. On the other hand, after taking his/her hands off the positioner 1, the operator can more easily form the horizontal tunnel 42 by manipulating a bone drill or another tool.

Optionally, the turning wheel 171 may be rotatably disposed at a proximal end of the handle 16 and restricted so as to displace in an axial direction of the handle 16. Since the handle 16 and the locating rod sleeve 15 are maintained stationary relative to each other once they are assembled together, the handle 16 can restrict the position of the turning wheel 171 with respect to the axial direction of the vertical locating rod 12. In the exemplary implementation shown in Fig. 10, the turning wheel 171 includes a turning wheel body 1710, a rotating shaft 1711 and a stopper 1712. The rotating shaft 1711 extends from a distal end of the turning wheel body 1710 along an axial direction thereof, and the stopper 1712 is provided at a distal end of the rotating shaft 1711. An outer diameter of the rotating shaft 1711 is smaller than an outer radial dimension of the stopper 1712 and than an outer radial dimension of the turning wheel body 1710. The handle 16 defines, at its proximal end, a corresponding rotating shaft accommodating ring 162 having an inner diameter matching the outer diameter of the rotating shaft 1711. The rotating shaft 1711 is rotatably inserted in the rotating shaft accommodating ring 162, and two end faces of the rotating shaft accommodating ring 162 are brought into abutment respectively with the turning wheel body 1710 and the stopper 1712. With this configuration, the handle 16 can restrict the axial position of the turning wheel 171 while allowing its free circumferential rotation. Of course, the above-discussed exemplary implementation is merely a preferred example of the turning wheel 171, and is not intended to limit the turning wheel 171 in any sense. Those skilled in the art can make various modifications and variations to it without departing the scope of the above teachings.

Optionally, a lead pitch of the turning wheel 171 and the push member 172 enables self-locking. That is, the ratio of the lead pitch to a diameter of the push member 172 is within a predetermined range. With this configuration, the horizontal locating rod 11 will be caused to move forth and back by rotating the turning wheel 171, while the turning wheel 171 will not rotate as a result of either advancement or retraction of the horizontal locating rod 11. That is, self-locking can be achieved. In practice, the lead pitch may be determined by the operator according to his/her habit of using rotary mechanical actuators. In an exemplary implementation, the turning wheel 171 may be configured so that the horizontal locating rod 11 can be sufficiently extended and come into abutment with the object 4 of interest when the turning wheel 171 is turned 1.5 or more revolutions but no more than 2 revolutions. Preferably, the push member 172 is secured to the proximal end of the horizontal locating rod 11 and defines a bore extending therethrough along its axial direction, which communicates with the horizontal channel 110 to allow passage of the conveyor shaft 21, a bone drill or another component therethrough.

Optionally, the horizontal locating rod 11 may have an abutment surface 111 at its distal end, which is complementary in shape to the object 4 of interest and adapted to come into abutment with the surface of the object 4 of interest. Additionally, the horizontal locating rod 11 may be provided at the distal end with an engagement unit 112 for piercing the surface of the object 4 of interest and thereby engaging the object 4 of interest. For example, the engagement unit 112 may be a sharp projection provided at an edge of the abutment surface 111. During use, after the abutment surface 111 is brought into abutment with the surface of the object 4 of interest, the turning wheel 171 may be slightly turned to cause the engagement unit 112 to slightly engaged into the bone. This allows the positioner 1 to more securely anchor to the object 4 of interest, ensuring that the subsequent formation of the horizontal tunnel 42 will not be affected by external vibration and achieve accurate locating.

Referring to Figs. 7 and 8, preferably, the conveyor 2 further includes a suture accommodating unit 23, which is coupled to a proximal end of the conveyor shaft 21 and adapted to accommodate a proximal end portion of the suture 32, for example, in the form of a spool. In the exemplary implementation shown in Figs. 7 and 8, the suture accommodating unit 23 includes a suture bobbin 231 and a case 232. The case 232 is attached to the proximal end of the conveyor shaft 21 and defines an inner cavity in communication with the suture cavity 210. The suture bobbin 231 is rotatably disposed within the case 232, and the suture 32 extends from the suture cavity 210 into the inner cavity of the case 232 and is wound on the suture bobbin 231. In some implementations, as a result of the suture 32 being pulled distally, the suture bobbin 231 can rotate about its own axis, allowing the suture 32 wound thereon to be distally pulled out. In some other implementations, after rotating a predetermined angle about its own axis, the suture bobbin 231 may be restricted by the case 232 from further rotation. At this time, when the suture 32 wound on the bobbin is further pulled distally, it will slide relative to the suture bobbin 231.

Referring to Fig. 11, in an alternative implementation, the conveyor tip 22 includes a retaining fork. A distal end of the retaining fork includes a locating retainer 251 and at least two fork prongs 252. The locating retainer 251 connects the at least two fork prongs 252 to form a closed retaining slot 250 for catching and retaining the coil 31 of the suture 32 or for accommodating the implant. The conveyor tip 22 may include two such retaining forks disposed in opposition to each other. The retaining slots 250 of the retaining forks may be utilized to retain a plate-shaped element of some implant (e.g., a titanium suture button), thus achieving retention of the entire implant. During use, the implant may be delivered into the horizontal tunnel 42 through the horizontal channel 110. Further, hooking and retention of the implant or suture 32 can be achieved by passing the hooking unit 131 through a gap between the two retaining forks.

Optionally, the handle 16, the turning wheel 171, the drive unit 14 and other components may be made of plastic materials for medical use. Preferred examples of such plastic materials include polyvinyl chloride (PVC), acrylonitrile butadiene styrene (ABS), polyethylene (PE), polypropylene (PP), polycarbonate, etc. The horizontal locating rod 11, the vertical locating rod 12, the locating rod sleeve 15 and other components may be made of metals for medical use, which may be independently selected from 12Cr13, 20Cr13, 05Cr17NiCu4Nb, 32Cr13Mo, 40Cr13, Y10Cr17, 06Cr19Ni10 and the like. Of course, the above-enumerated materials are merely examples, and the components are not limited to being made of them. Those skilled in the arts may select any other suitable known prior materials.

Preferably, the conveyor 2 further includes a stop unit 24 fixedly attached to the conveyor shaft 21. The stop unit 24 is adapted to come into abutmemt with a corresponding portion of the positioner 1, thereby disallowing further distal displacement of the conveyor shaft 21 relative to the horizontal channel 110. At the time when the stop unit 24 comes into abutmemt with the corresponding portion of the positioner 1, the conveyor tip 22 is located at the intersection of the direction of extension of the distal end of the vertical channel 120 and the direction of extension of the distal end of the horizontal channel 110. In the exemplary implementation shown in Fig. 7, the joint of the case 232 and the conveyor shaft 21 may serve as the stop unit 24, and the proximal end of the handle 16 or the turning wheel 171 in the positioner 1 may provide the corresponding portion that the stop unit 24 is to be brought into abutment with. As shown in Figs. 1 and 2, after the conveyor shaft 21 of the conveyor 2 is inserted into the horizontal channel 110, during its distal movement therein, upon the stop unit 24 coming into abutment with the turning wheel 171, it may be not impossible to distally advance the conveyor 2 any further. Of course, in some other implementations, the stop unit 24 and the corresponding portion of the positioner 1 may be provided by a pair of corresponding separate elements, such as a stop boss and a stop ring. Those skilled in the art may properly configure them as practically needed, and the present invention is limited to in this regard.

Optionally, according to this embodiment, the conveyor 2 may be a disposable product, and a single suture 32 may be placed therein beforehand. Depending on the requirements of an intended tendon repair procedure, the suture 32 may be round, flat or oblong (rounded at both ends and flat in the middle). The suture 32 may be formed of natural fiber, synthetic fiber, a metal wire or the like. Examples of the natural fiber may include silk and the like. Examples of the synthetic fiber may include extra-high molecular weight polyethylene, polyamide 6/6, polypropylene, polyethylene terephthalate, polyamide 6 and the like. A round suture of a size between 3-0 and 5, or a flat suture of 0.1 mm to 5 mm may be included in advance. In contrast, the positioner 1 may be reused many times. After the suture 32 that is included in advance has been passed through the tunnel, the operator may discard the used conveyor 2 and if necessary, deploy the suture of a new conveyor 2.

A operating method of the above-discussed system is described below in the context of the object 4 of interest being a humerus. The method includes the steps as follows.

Step S1: Forming a vertical tunnel 41 in the humerus. As there is little muscle where the vertical tunnel 41 is to be formed, the operator can fast and accurately identify a suitable location for the vertical tunnel 41 by palpation and then form the vertical tunnel 41 with a broach or drill.

Step S2: Inserting the distal end of the vertical locating rod 12 of the positioner 1 into the vertical tunnel 41 so that the direction of extension of the distal end of the vertical channel 120 coincides with the axial direction of the vertical tunnel 41. Optionally, after step S2 is complete, the method may include step S21: using the locking unit 17 to drive the horizontal locating rod 11 to distally move so that the horizontal locating rod 11 clamps the humerus together with the vertical locating rod 12. In this way, the positioner 1 anchors to the humerus. For example, the turning wheel 171 may be turned to bring the distal end of the horizontal locating rod 11 into abutment with the greater tubercle of the humerus. After that, the turning wheel 171 may be slightly rotated to cause the engagement unit 112 to slightly engaged into the humerus. As a result, the vertical locating rod 12, the horizontal locating rod 11 and the humerus firmly abut and lock against one another.

Step S3: Forming a horizontal tunnel 42 in the humerus along the direction of extension of the horizontal channel 110 in the positioner 1, which intersects the vertical tunnel 41. In an exemplary implementation, this step can be carried out using a bone drill. Specifically, the bone drill may be extended out of the horizontal channel 110 from the distal end thereof and operated to form the horizontal tunnel 42 along the direction of extension of the horizontal channel 110. The bone drill may have a helical groove, through which bone debris (including that produced during the formation of the vertical tunnel 41) can be discharged during rotation of the drill, avoiding occlusion of the bone tunnels. The bone drill may reach a depth required by the horizontal tunnel 42 and the vertical tunnel 41 to intersect each other. Preferably, in practice, the bone drill may be provided with a stop unit which can prevent drill from extending too deep into the bone. After the formation of the horizontal tunnel 42 is completed, the bone drill is withdrawn.

Step S4: Inserting the conveyor shaft 21 of the conveyor 2 into the horizontal channel 110 so that the distal end of the conveyor shaft 21 enters the horizontal tunnel 42 and that the conveyor 2 is located at the intersection of the vertical tunnel 41 and the horizontal tunnel 42. Before or after step S4, the push button 141 of the drive unit 14 may be optionally distally pushed to extend out the connecting unit. Upon the conveyor shaft 21 of the conveyor 2 being distally moved into place (e.g., when the stop unit 24 comes into abutment with the positioner 1), the connecting unit will tie the coil 31 in the conveyor tip 22 (e.g., it is hooked by the hooking unit 131).

Step S5: Using the drive unit 14 to drive the connecting unit to move proximally so that it catches the coil 31 or the implant accommodated in the conveyor tip 22. Optionally, after this step, the conveyor 2 may be gently pulled back proximally. If resistance is perceived, it can be known that the suture has been successfully caught.

Step S6: Proximally pulling and withdrawing the conveyor 2 so that the coil 31 or the implant remains in the vertical tunnel 41. In this step, the conveyor 2 may be pulled little more strongly until the suture 32 or implant is completely detached from the conveyor 2.

Step S7: Proximally pulling the positioner 1 so that part of the coil 31 or the implant (e.g., a tensioning suture of the implant, etc.) is pulled out of the vertical tunnel 41. Optionally, before step S7, the positioner 1 may be unlocked from the humerus by rotating the turning wheel 171 in the opposite direction.

Step S8: Extending out the connecting unit by distally pushing the push button 141 and detaching the coil 31 or the implant from the connecting unit. Once the coil 31 or the implant is separated from the positioner 1, its passage through the bone tunnels is completed.

### Embodiment 2

In a second embodiment of the present invention, there are provided a positioner, a conveyor, a tunnel-type wire passing system and an operating method thereof, which are substantially similar to those of the first embodiment. Only differences of this embodiment from the first embodiment will be described below, and any feature it commonly shares with the first embodiment will not be described again.

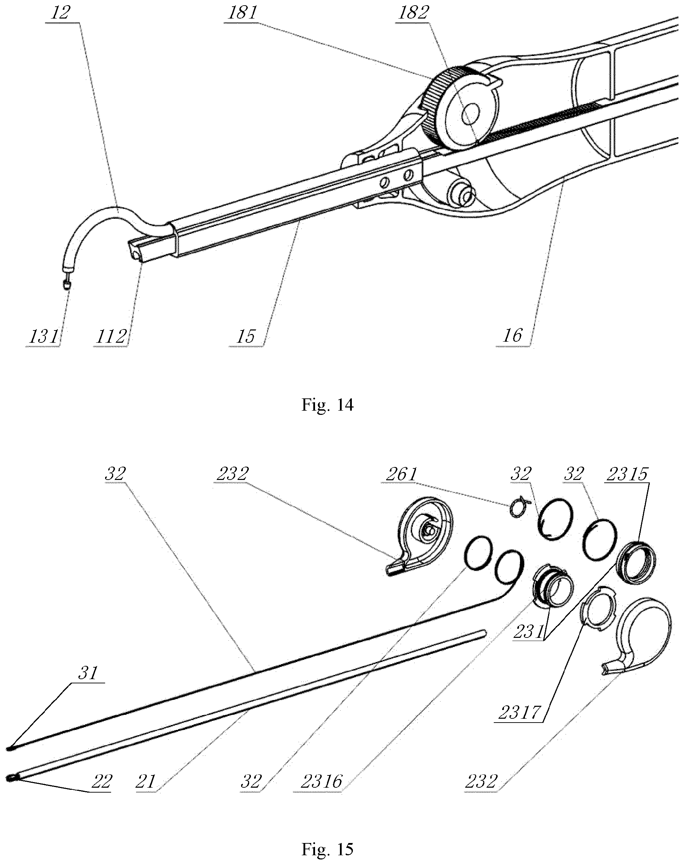

Reference is now made to Figs. 12 to 14. Fig. 12 is a schematic illustration of a vertical locating rod according to the second embodiment of the present invention. Fig. 13 is a schematic illustration of a first rotating wheel according to the second embodiment of the present invention. Fig. 14 is a schematic illustration of a second rotating wheel and a toothed rack according to the second embodiment of the present invention.

The positioner 1 according to the present second embodiment differs in structure from that of the first embodiment. Specifically, as shown in Fig. 12, in the positioner 1, at least part of the vertical locating rod 12 is made of a shape memory material, such as a shape memory metal whose strength varies with temperature. Depending on needs, the vertical locating rod 12 may be preformed in real time to have various angles or shapes which uniquely conform to specific objects 4 of interest. In some implementations, the proximal end of the vertical locating rod 12 is fixedly attached to the vertical locating rod accommodating bore 151 of the locating rod sleeve 15. In some alternative implementations, the vertical locating rod 12 may be detachable from the locating rod sleeve 15. In use, the vertical locating rod 12 may be assembled and reliably engaged in the vertical locating rod accommodating bore 151 of the locating rod sleeve 15 by an interference fit or another form of engagement. Once the vertical locating rod 12 is inserted into the vertical locating rod accommodating bore 151, they can tightly engage each other, facilitating subsequent locking of the positioner 1 by the locking unit 17.

Preferably, the curved section 121 of the vertical locating rod 12 is made of a shape memory material, while the straight section 122 may be made of a shape memory material, or preferably of a regular non-shape memory material.

Optionally, the vertical locating rod 12 is adapted to be softened in an environment at a first predetermined temperature, bent into a shape in conformity with an object 4 of interest and hardened at a second predetermined temperature. The first and second predetermined temperatures may be determined according to an environment of use. In an exemplary implementation, the first predetermined temperature may be 60 °C, for example, and the second predetermined temperature may be 40 °C, for example. The shape memory material may become soft and reshapable at 60 °C or above, and may become hard and non-reshapable and restore its original strength and hardness when cooling to 40 °C or below. In an exemplary implementation, an operator may soften the shape memory part of the vertical locating rod 12 by submerging it in hot water, and bend it in accordance with the shape of the object 4 of interest, in particular the shape of a vertical tunnel 41 preformed therein. After that, the vertical locating rod 12 may be cooled naturally or in cold water to assume the desired curved shape. Of course, the cold water and the hot water are merely an example of means for providing the predetermined temperatures, and those skilled in the art may also achieve the predetermined temperatures using, for example, a heat gun or an oven.

According to the second embodiment, the drive unit 14 is structured differently from that of the first embodiment. Specifically, referring to Fig. 13, the drive unit 14 includes a first rotating wheel 142, and the proximal end of the guide wire 13 is fixedly coupled to the first rotating wheel 142 by a connecting link 132. The guide wire 13 is adapted to move forth and back along its own axial direction as a result of rotation of the first rotating wheel 142.

Referring to Fig. 14, preferably, the positioner 1 further includes a second rotating wheel 181 and a toothed rack 182. The second rotating wheel 181 has a toothed outer circumference, and the toothed rack 182 is fixed connected to the horizontal locating rod 11. The toothed rack 182 is adapted to engage the toothed outer circumference. Rotation of the second rotating wheel 181 can be converted, by the toothed rack 182 and the toothed outer circumference in engagement therewith, into movement of the horizontal locating rod 11 along its own axial direction.

In an alternative implementation, the first rotating wheel 142 includes two portions disposed in opposition to each other. The two portions may be disposed on opposing sides of the second rotating wheel 181 along an axial direction thereof in such a manner that the first rotating wheel 142 and the second rotating wheel 181 can rotate independently of each other. Optionally, stop unit may be provided to ensure independent movability of the two, i.e., to prevent the first rotating wheel from following the second rotating wheel to rotate, and to prevent the second rotating wheel from following the first rotating wheel to rotate. When the second rotating wheel 181 is rotated in one direction, it may drive the horizontal locating rod 11 to move distally. For ease of description, the direction of rotation of the second rotating wheel 181 which causes distal movement of the horizontal locating rod 11 is referred to hereinafter as a direction of advancement (clockwise in Fig. 14). Thus, when the second rotating wheel 181 is rotated in the direction of advancement, the horizontal locating rod 11 will be driven to move distally, while the first rotating wheel 142 will stay stationary without causing the guide wire 13 to extend out distally. Moreover, once the horizontal locating rod 11 is locked by the locking unit 17 and cannot move, the second rotating wheel 181 will be consequently locked and cannot rotate. At this time, when the first rotating wheel 142 is rotated in the direction of advancement (clockwise in Fig. 14), the connecting link 132 will drive the guide wire 13 to extend out distally.

In correspondence to the above-discussed positioner 1 of the second embodiment, the method of the second embodiment further includes, prior to step S2:
Step S20: softening at least part of the vertical locating rod 12 by placing it in an environment at a first predetermined temperature, bending the vertical locating rod 12 into a shape in conformity with that of the object 4 of interest, and hardening the at least part of the vertical locating rod 12 by placing it at a second predetermined temperature.

Moreover, in step S21, the horizontal locating rod 11 may be caused to move distally by rotating the second rotating wheel 181. Further, it will be appreciated that, in steps S4 to S8, the guide wire 13 may be driven to advance or retract by rotating the first rotating wheel 142.

### Embodiment 3

In a third embodiment of the present invention, there are provided a positioner, a conveyor, a tunnel-type wire passing system and an operating method thereof, which are substantially similar to those of the first embodiment. Only differences of this embodiment from the first embodiment will be described below, and any feature it commonly shares with the first embodiment will not be described again.

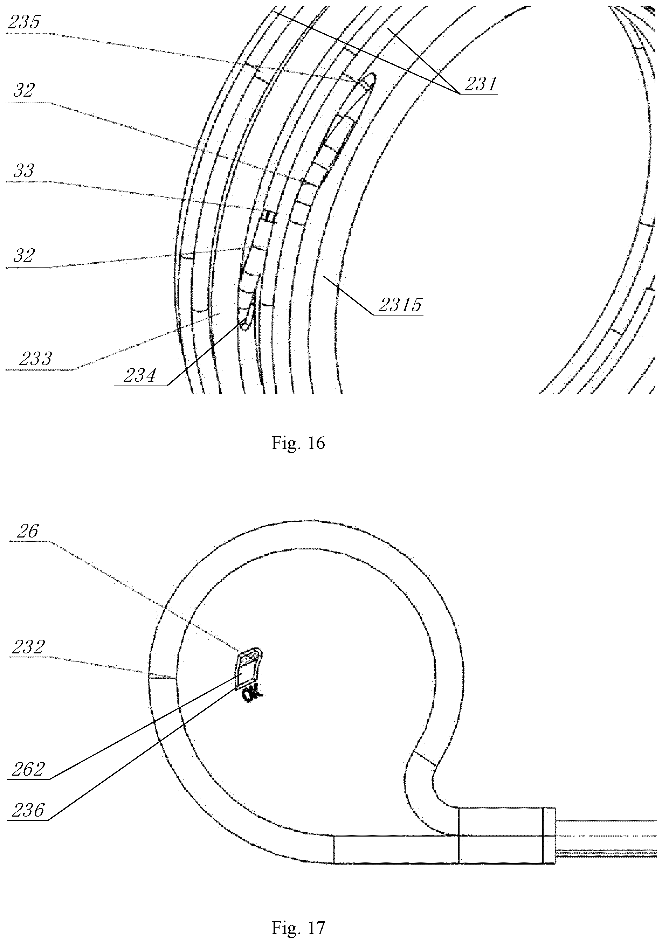

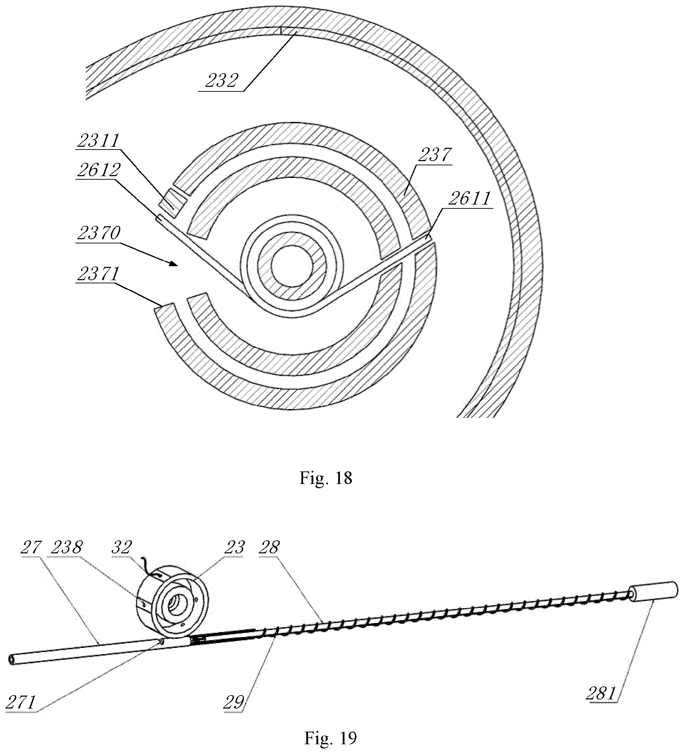

Reference is now made to Figs. 15 to 18. Fig. 15 is a schematic exploded view of the conveyor according to the third embodiment of the present invention. Fig. 16 is a schematic illustration of an outer suture bobbin group according to the third embodiment of the present invention. Fig. 17 is a schematic illustration of an indication unit according to the third embodiment of the present invention. Fig. 18 is a schematic illustration of a torsion spring according to the third embodiment of the present invention.

The conveyor 2 according to the present third embodiment differs in structure from that of the first embodiment. Specifically, as shown in Fig. 15, the conveyor 2 includes a conveyor shaft 21, a conveyor tip 22 and at least two suture bobbins 231. Each of the suture bobbins 231 is adapted for winding of a single suture 32 thereon. The conveyor shaft 21 defines a suture cavity 210 extending along its axial direction. The suture cavity 210 is adapted to accommodate distal extensions of the sutures 32 wound on the suture bobbins 231. The conveyor tip 22 is provided at a distal end of the conveyor shaft 21 and adapted to accommodate coils 31 formed by portions of the sutures 32 that distally extend out of the suture cavity 210. The conveyor shaft 21 is adapted to be movably inserted in the positioner 1, and the conveyor tip 22 is adapted to protrude out of the positioner 1 from its distal end so that the coils 31 accommodated in the conveyor tip 22 can be hooked and retained by the positioner 1.

Multiple sutures are often used in a single surgical procedure. With the above configuration, at least two sutures 32 can be included in the conveyor 2. After one suture 32 is passed through a tunnel, the conveyor 2 can continue to be used to pass another suture 32 through the tunnel, without replacement with a new conveyor 2. This can improve continuity of the surgical procedure and reduce total cost of conveyor(s) 2 used therein.

On the basis of the conveyor 2, in this embodiment, there is also provided a suture passing instrument including the conveyor 2 and at least two sutures 32 each wound on a respective one of the suture bobbins 231 in the conveyor 2. Sutures 32 wound on adjacent suture bobbins 231 are tied end-to-end. One of the sutures 32 is partially accommodated in the suture cavity 210 of the conveyor 2 and is distally wound into a coil 31 accommodated in the conveyor tip 22 of the conveyor 2. In practice, at least two identical or different sutures 32 may be tied end-to-end, and each of them may be formed at the tip end into a coil 31. It would be appreciated that the coils may be preformed before the sutures 32 are delivered from the factory, or formed intraoperatively by an operator. Integrating the sutures 32, the suture passing instrument is adapted to passing the sutures 32 into a horizontal tunnel 42 formed in an object 4 of interest. It is to be noted that the present invention is not limited to how the sutures 32 wound on the suture bobbins 231 are (proximally) attached to the suture bobbins 231. In a preferred implementation, the sutures 32 are only wound on the suture bobbins 231, without being fixed thereto. With this configuration, the sutures 32 can slide relative to the suture bobbins 231 and can be distally pulled out when the suture bobbins 231 are fixed.

As shown in Fig. 16, optionally, the suture passing instrument may further include suture tie(s) 33 connected between adjacent sutures 32. Tensile strength of the suture tie(s) 33 is lower than that of the sutures 32. Preferably, each suture tie 33 may be weakened and made of the same material as the sutures 32. The weakening may be accomplished by partial removal of the material. Thus, the tie(s) can be easily pulled apart by the operator during surgery. Based on statistics of general amounts used in surgical procedures, each suture 32 may be set to 1 meter long, for example. Of course, in some alternative implementations, each suture tie 33 may be made of a material different from and with lower tensile strength than that of the sutures 32.

Referring to Fig. 16, in one implementation, the at least two suture bobbins 231 are arranged side by side along an axis. The at least two sutures 32 that are tied end-to-end are sequentially wound on the respective suture bobbins 231. Preferably, all the sutures 32 are wound in the same direction. Juxtaposing the at least two suture bobbins 231 along the axis can separate the sutures 32 apart from one another and avoid intraoperative interference and tangling of them.

Preferably, the conveyor 2 includes suture spacer(s) 233 interposed between adjacent suture bobbins 231 along the axial direction and adapted to separate the sutures 32 on the respective suture bobbins 231 from one another. The suture spacer(s) 233 may be ridge-like protrusion(s) with an outer diameter greater than that of the suture bobbins 231. Thus, it/they can separate the sutures 32 on the respective suture bobbins 231 from one another.

Additionally, each suture spacer 233 defines a first suture slot 234 adapted for passage of a suture 23 therethrough. In this way, the sutures 32 on the suture bobbins 231 that are juxtaposed along the axial direction can be tired together. Preferably, the first suture slot(s) 234 extend(s) obliquely through the suture spacer(s) 233, allowing the sutures 32 to be more easily pulled out from the suture bobbins 231. It will be appreciated that in each first suture slot 234 may be inserted a suture tie 33 or either of the sutures 32 separated by the suture spacer 233. In the latter case, suture tie(s) 33 may be located on one side of the first suture slot(s) 234.

With continued reference to Fig. 15, in an alternative implementation, the at least two suture bobbins 231 nest within one another around an axis. The above-described configuration in which the suture bobbins 231 are juxtaposed along the axial direction is limited in the number of sutures 32 because it will suffer from an excessively large aggregate width of the suture bobbins 231 when the number of sutures 32 is relatively large. In order to overcome this, the at least two suture bobbins 231 may be configured to nest within one another around an axis. In this case, at least one suture 32 may be wound on each suture bobbin 231. Preferably, the two configurations may be combined, i.e., the suture bobbins 231 are arranged into groups juxtaposed along the axis, each of which consists of suture bobbins 231 nesting within one another around the axis. In an alternative exemplary implementation, two suture bobbins 231 are juxtaposed along an axis to form an outer suture bobbin group 2315, and other two suture bobbins 231 are also juxtaposed along the axis to form an inner suture bobbin group 2316. Thus, the outer suture bobbin group 2315 surrounds the inner suture bobbin group 2316 around the axis. The outer suture bobbin group 2315 and the inner suture bobbin group 2316 may be configured to be rotatable independently of each other. Optionally, the inner suture bobbin group 2316 may cooperate with a stop plate 2317 to prevent dislodgement of the outer suture bobbin group 2315.

Preferably, the two suture bobbins 231 in the outer suture bobbin group 2315 are secured to each other, and the two suture bobbins 231 in the inner suture bobbin group 2316 are also secured to each other. With this configuration, four sutures 32 can be accommodated in the conveyor 2. It will be appreciated that those skilled in the art may appropriately modify the number and arrangement of the suture bobbins 231 in light of the above teachings. For example, in order to meet the demand of some practical applications, suture bobbins may be arranged into two layers, each containing three juxtaposed suture bobbins.

With continued reference to Fig. 16, preferably, outer suture bobbins 231 define second suture slots 235 for passage of sutures 32 therethrough. In this way, sutures 32 on radially adjacent suture bobbins 231 are tied together. In an exemplary implementation, one suture bobbin 231 in an outer suture bobbin group may define a second suture slot 235. In this case, sutures 32 may be wound successively on suture bobbins 231 in the outer suture bobbin group, passed inwardly through the second suture slot 235 from the outer suture bobbin group, and successively on suture bobbins 231 in an inner suture bobbin group. With this configuration, the sutures 32 wound on the inner and outer suture bobbins may be tied together to form a continuous line and can be easily pulled out successively for use during surgery. It is to be noted that, here, any outer suture bobbin 231 is not necessarily in an outermost one of suture bobbin groups arranged in layers. Rather, it should be considered as one surrounding another suture bobbin. That is, it should be considered as not belonging to an innermost suture bobbin group. For example, when the conveyor 2 has suture bobbin groups arranged into three layers, the intermediate suture bobbin group can be taken as an outer group of the innermost one. Therefore, second suture slot(s) 235 may be arranged in the intermediate suture bobbin group to enable sutures 32 to successively wound respective suture bobbins. Preferably, in order to avoid tangling of sutures 32, a second suture slot 235 is provided in a laterally outermost suture bobbin 231 in each suture bobbin group.

Referring to Fig. 17, optionally, the conveyor 2 may include an indication unit 26. When one suture bobbin 231 is rotating under the drive of a suture 32, one end of the indication unit 26 where it is coupled to the suture bobbin 231 will be caused to displace relative to the case 232, thereby providing an indication. The inventors have found that the bone tunnels (i.e., the vertical and horizontal tunnels 41, 42 formed in the object 4 of interest) are blind zones, and their insides are invisible to the operator. Therefore, he/she usually has to totally rely on hand sensation and experience to determine conditions of an instrument inserted therein. This is, however, associated with a high surgical risk. In order to overcome this problem, the indication unit 26 is configured to provide, to the operator, on the conveyor 2 outside of the bone tunnels, an indication of whether the suture 32 has been successfully hooked and retained by the hooking unit 131.

Referring to Figs. 17 and 18, preferably, the indication unit 26 includes a torsion spring 261 and an indication mark 262. One end of the torsion spring 261 is coupled to the suture bobbin 231, and the other end is coupled to the case 232. The indication mark 262 is attached to the end of the torsion spring 261 where it is coupled to the suture bobbin 231. The case 232 has an observation area 236 allowing observation of the indication mark 262.

In an exemplary implementation, the case 232 defines a suture bobbin axle 237. A first end 2611 of the torsion spring 261 is embedded in the suture bobbin axle 237 and restricted thereby from rotation about the suture bobbin axle 237. A second end 2612 of the torsion spring 261 is disposed in a circumferential opening 2370 of the suture bobbin axle 237 so as to be moveable therein. When not subject to an external force, the second end 2612 of the torsion spring 261 abuts against one edge of the circumferential opening 2370 (the upper edge in Fig. 18) due to the spring's own resilience. The suture bobbin 231 is rotatably disposed around the suture bobbin axle 237 and defines a push post 2311 for abutment and engagement of the suture bobbin 231 with the second end 2612 of the torsion spring 261. Once the suture 32 is successfully hooked and retained by the hooking unit 131, it will be distally pulled out by the hooking unit 131, causing rotation of the suture bobbin 231 about its own axis in a first direction (counterclockwise in Fig. 18) and hence displacement of the second end 2612 of the torsion spring 261 relative to the case 232. It is to be noted that the aforementioned first direction may be either of clockwise and counterclockwise directions, rather than being limited to being the counterclockwise direction as described in the above exemplary implementation. The indication mark 262 is attached to the second end 2612 of the torsion spring 261. As a result of the second end 2612 of the torsion spring 261 being pushed to move, the indication mark 262 displaces therewith relative to the case 232. The operator can observe the displacement of the indication mark 262 through the observation area 236. In an alternative implementation, the case 232 is substantially cylindrical in shape, and the observation area 236 is provided on one base of the cylinder, for example, in the form of a cutout optionally covered with a transparent material. As another example, said base or the entire cylindrical case 232 may be formed of a transparent material. An indication mark (e.g., "OK") may be displayed at a location of the case 232 near the observation area 236. Movement of the indication mark 262 into the observation area 236 indicates success in hooking and retaining the suture 32.

It will be appreciated that the above-described indication unit 26 is suitable for use in the case of the conveyor 2 including only a single suture 32, as in the first embodiment. In the case of the conveyor 2 including at least two sutures 32, the case 232 is preferred to have a rotation stop unit 2371 adapted to come into abutment with the push post 2311 or the torsion spring 261 after the suture bobbin 231 has rotated a predetermined angle, thereby preventing its further rotation in the first direction. With continued reference to Fig. 18, the rotation stop unit 2371 may be provided by an edge of the circumferential opening 2370 of the suture bobbin axle 237 on the side opposite to the edge that the second end 2612 of the torsion spring 261 comes into abutment with when it is not subject to an external force. In the exemplary implementation shown in Fig. 18, when the second end 2612 of the torsion spring 261 comes into abutment with the rotation stop unit 2371 as a result of being pushed by the push post 2311 to rotate in the first direction (counterclockwise) about an axis of the suture bobbin axle 237, the rotation stop unit 2371 will prevent the second end 2612 of the torsion spring 261 and the push post 2311, and hence the suture bobbin 231, from further rotating in the first direction. At this time, further pulling the suture 32 distally will cause it to slide relative to the suture bobbin 231. Meanwhile, the indication mark 262 still remains in the observation area 236, and the mark "OK" is displayed. After the current suture 32 is completely pulled out and separated from the next suture 32, it no longer exerts a torque on the suture bobbin 231 in the first direction, and the torsion spring 261 biases the suture bobbin 231 back to its initial position shown in Fig. 18. As a result, the indication mark 262 moves out of the observation area 236, and the mark "OK" is no longer displayed until the next suture is successfully caught. It will be appreciated that while the rotation stop unit 2371 has been described as being provided by part of the suture bobbin axle 237 in the foregoing exemplary implementation, it may alternatively be implemented as a separate component on the case 232 in other implementations, which may limit rotation of the suture bobbin 231 also by coming into abutment with the push post 2311. The present invention is not limited in this regard.

In correspondence to the above-discussed conveyor 2 and suture passing instrument, the method of this embodiment further includes, after step S9:
Step S91: further proximally pulling the current suture whose coil has been pulled out of the vertical tunnel 41 in step S7 until a distal end of the next suture that is tied to the current suture moves beyond the conveyor tip 22; and
Step S92: separating the current suture from the next suture, retaining the distal coil of the next suture within the conveyor tip 22 and repeating the suture passing process with the next suture on the object 4 of interest.

Likewise, the distal coil 31 of the next suture 32 may be preformed before the suture 32 is delivered from the factory, or formed at the site in step S92 by the operator.

### Embodiment 4

In a fourth embodiment of the present invention, there are provided a positioner, a conveyor, a tunnel-type wire passing system and an operating method thereof, which are substantially similar to those of the first embodiment. Only differences of this embodiment from the first embodiment will be described below, and any feature it commonly shares with the first embodiment will not be described again.

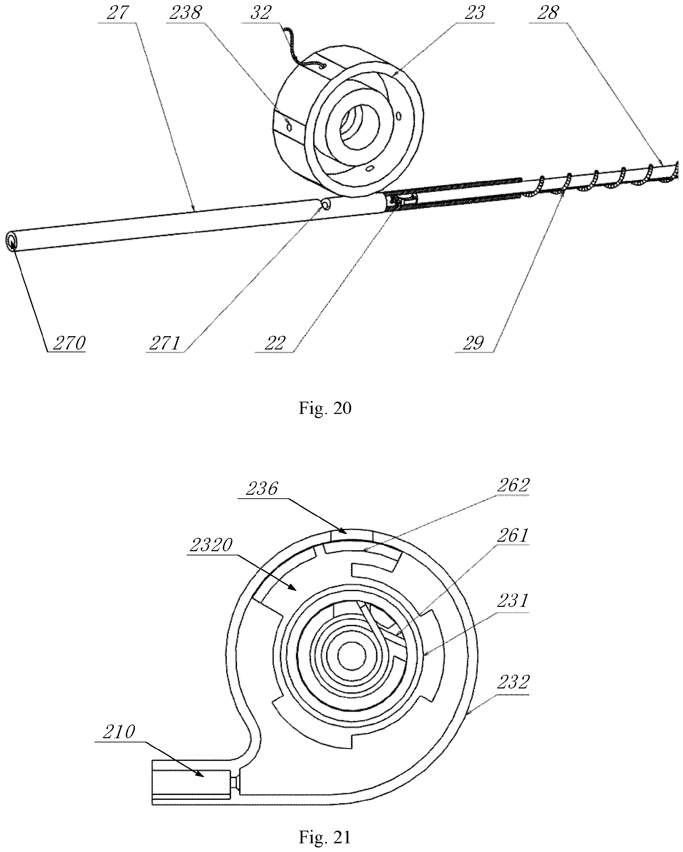

Reference is now made to Figs. 19 to 20. Fig. 19 is a schematic illustration of the conveyor according to the fourth embodiment of the present invention. Fig. 20 is an enlarged partial view of the conveyor of Fig. 19.

The conveyor 2 according to the present fourth embodiment differs in structure from that of the first embodiment. Specifically, as shown in Figs. 19 and 20, the conveyor 2 includes a sleeve 27, a push rod 28 and a conveyor tip 22. The sleeve 27 defines a push rod passage 270 extending therethrough along its axial direction and a radial opening 271 in communication with the push rod passage 270. Part of the push rod 28 is movably inserted in the push rod passage 270 along the axial direction of the sleeve 27. The conveyor tip 22 is coupled to a distal end of the push rod 28. The opening 271 is adapted for passage of a coil 31 of the suture 32 therethrough, and the push rod 28 is adapted to distally move from a location of the push rod passage 270 that is proximal with respect to the opening 271. The conveyor tip 22 is adapted to catch the coil 31 inserted into the push rod passage 270 through the opening 271 during the distal movement of the push rod 28 and protrude out of the sleeve 27 from its distal end. The sleeve 27 is adapted to be movably inserted in the positioner 1, and the conveyor tip 22 is adapted to protrude out of the positioner 1 from its distal end so that the positioner 1 can hook and retain the coil 31 accommodated in the conveyor tip 22.

With this configuration, after the coil 31 of the suture 32 is inserted into the push rod passage 270 through the opening 271, catching and retaining of the coil 31 at the conveyor tip 22 can be easily and conveniently achieved by distally moving the push rod 28 without using any additional tool. In this way, multiple sutures 32 can be consecutively deployed in a convenient way.

Optionally, the conveyor tip 22 may switch between a first state and a second state. In the first state, the conveyor tip 22 is inserted in the push rod passage 270 and restricted by the sleeve 27. After the conveyor tip 22 is pushed out of the sleeve 27 from its distal end, it will radially expand with respect to the sleeve 27 into the second state, thus tensioning the coil 31. In an alternative implementation, the conveyor tip 22 is made of a shape memory material, which can deform in a reversible manner. Thus, when received in the push rod passage 270 and restricted by the sleeve 27, the conveyor tip 22 will spontaneously shrink into conformity with an inner diameter of the push rod passage 270 and assume the first state. Moreover, when pushed out of the sleeve 27, the conveyor tip 22 will spontaneously expand into the second state.

In another optional implementation, the conveyor tip 22 has radial resilience with respect to the sleeve 27. When inserted in the push rod passage 270, the conveyor tip 22 is in the first state where it is restricted by the sleeve 27 and stores resilience energy. Once pushed out of the sleeve 27 from its distal end, the conveyor tip 22 releases the stored resilience energy and expands radially with respect to the sleeve 27 into the second state to tension the coil 31.

Preferably, as shown in Fig. 11, the conveyor tip 22 has a sloped guide surface 223 which is inwardly inclined toward an axis of the push rod 28 in the direction from distal to proximal. With the sloped guide surface 223, during proximal retraction of the push rod 28 into the sleeve 27, the sleeve 27 will inwardly compress the conveyor tip 22, facilitating switching of the conveyor tip 22 from the second state to the first state.

With continued reference to Fig. 19, optionally, the conveyor 2 may include a retraction drive unit 29 attached to the push rod 28. The retraction drive unit 29 is configured to store energy (because of being stretched) during proximal movement of the push rod 28 caused by a pulling external force and release the stored energy upon removal of the external force on the push rod 28 and thereby drive the push rod 28 to move distally until the conveyor tip 22 reaches the distal end of the sleeve 27 and protrudes out of the push rod passage 270 from its distal end. With the retraction drive unit 29, the push rod 28 can automatically retract to the distal end of the push rod passage 270 when it is not subject to an external force, enabling consecutive distal delivery of sutures 32 and allowing suture catching to be achieved with only one hand (without needing to control the conveyor 2 by hand). In some implementations, the retraction drive unit 29 includes an elastic member which is coupled at one end to the push rod 28 and at the other end to the sleeve 27. Of course, in some other implementations, the retraction drive unit 29 may include, for example, a magnetic component capable of storing and releasing energy in a magnetic manner.

Referring to Fig. 19, in an alternative exemplary implementation, the retraction drive unit 29 includes an elastic member which is a spring disposed over the push rod 28. The push rod 28 has a proximal expanded section 281, and the spring is brought into abutment at one end with the expanded section 281 and at the other end with a proximal end of the sleeve 27. When the push rod 28 is not subject to an external force, the conveyor tip 22 of the push rod 28 is biased by the spring and thereby located at the distal end of the push rod passage 270. Preferably, between the push rod 28 and the sleeve 27, there are provided step means, for example, in the form of mating snap fasteners, which facilitate use by preventing proximal dislodgement of the push rod 28 from the sleeve 27.

Optionally, the conveyor 2 may further include a suture accommodating unit 23 adapted to accommodate at least two sutures 32. The suture accommodating unit 23 defines at least two suture holes 238 each adapted for pull-out of a respective one of the sutures 32 therethrough. The at least two suture holes 238 are adapted for successive alignment with the opening 271. This embodiment is not limited to any particular structure of the suture accommodating unit 23. For example, it may include suture bobbins nesting within one another as described in the foregoing third embodiment, axially juxtaposed suture bobbins, or the like. Preferably, the at least two suture holes 238 are uniformly spaced circumferentially around the suture accommodating unit 23. In the exemplary implementation shown in Fig. 20, the suture accommodating unit 23 has six suture holes 238 and accommodates six sutures 32 each having a distal end portion which has been pulled out of a respective one of the six suture holes 238, hardened with glue or otherwise and shaped into a coil 31 oriented at an angle in conformity with the opening 271. In this way, the coils 31 can be easily introduced into the push rod passage 270 through the opening 271. After one suture 32 is pushed out of the sleeve 27 with the push rod 28, the suture accommodating unit 23 may be turned to align the next suture hole 238 with the opening 271, allowing the delivery of the next suture 32. Without limitation, the suture accommodating unit 23 may be turned, either manually, or in an automated manner using a mechanical mechanism.

Preferably, in this embodiment, in order to facilitate catching of the coils 31, the conveyor tip 22 may optionally adopt a design with two retaining forks disposed in opposition, as described in the first embodiment. Reference can be made to Fig. 11 or the first embodiment for more details in this regard.

On the basis of the above-discussed conveyor 2, this embodiment also provides a suture passing instrument including the conveyor 2 as defined above and at least two sutures 32. The at least two sutures 32 have wound portions in the form of coils 31 which are adapted to be successively introduced into the push rod passage 270 through the opening 271 so that the conveyor tip 22 can successively catch and push them out of the sleeve 27 from its distal end. Correspondingly, the system of this embodiment includes this suture passing instrument and the positioner 1. After a coil 31 is caught and pushed by the conveyor tip 22 of the conveyor 2 out of the sleeve 27 from its distal end, the sleeve 27 is inserted into the horizontal channel 110 of the positioner 1, and the connecting unit of the positioner 1 is then tied to the coil 31 retained at the conveyor tip 22 of the conveyor 2.

Correspondingly, the method of this embodiment further includes, prior to step S4:
Step SA: inserting the coil 31 of the suture 32 into the push rod passage 270 through the opening 271 of the conveyor 2 and distally pushing the push rod 28 so that the conveyor tip 22 catches the coil 31 and pushes it out of the sleeve 27 from its distal end.

After step S5, the method further includes the steps as follows:
Step SB1: moving the sleeve 27 proximally to withdraw it from the horizontal channel 110.

Step SB2: inserting the coil 31 of the next suture 32 into the push rod passage 270 through the opening 271 of the conveyor 2, distally pushing the push rod 28 so that the conveyor tip 22 catches the coil 31 of the next suture 32 and pushes it out of the sleeve 27 from its distal end, and repeat the suture passing process on the object 4 of interest. Optionally, before or after the coil 31 of the next suture 32 is inserted into the push rod passage 270 through the opening 271 of the conveyor 2, the method may further include step SB3: proximally moving the push rod 28 so that the conveyor tip 22 is located proximally with respect to the opening 271.

The conveyor 2 and the suture passing instrument of this embodiment allow automatic retraction and catching of the next suture 32. This can enhance continuous progress of a surgical procedure and simplify the retention of the coil 31 of a suture 32 at the conveyor tip 22.

### Embodiment 5

In a fifth embodiment of the present invention, there are provided a positioner, a conveyor, a tunnel-type wire passing system and an operating method thereof, which are substantially similar to those of the first embodiment. Only differences of this embodiment from the first embodiment will be described below, and any feature it commonly shares with the first embodiment will not be described again.

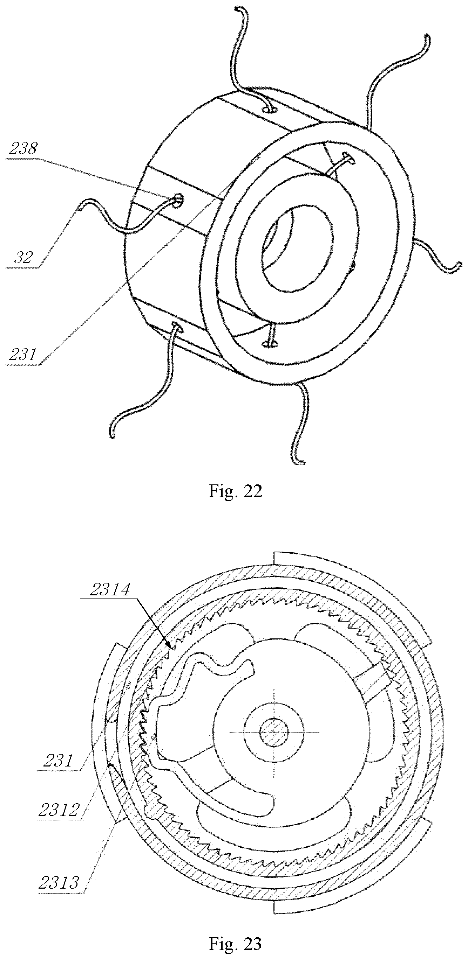

Reference is now made to Figs. 21 to 23. Fig. 21 is a schematic partial view of the conveyor according to the fifth embodiment of the present invention. Fig. 22 is a schematic overview of a suture accommodating unit according to the fifth embodiment of the present invention. Fig. 23 schematically illustrates the internal structure of the suture accommodating unit according to the fifth embodiment of the present invention.

The conveyor 2 according to the present fifth embodiment differs in structure from that of the first embodiment. Specifically, as shown in Figs. 21 and 22, the conveyor 2 includes a conveyor shaft 21, a conveyor tip 22 and a suture accommodating unit 23. The suture accommodating unit 23 is joined to the conveyor shaft 21 and adapted to accommodate at least two sutures 32. The suture accommodating unit 23 defines at least two suture holes 238 each adapted for pull-out of a respective one of the sutures 32 therethrough. The at least two suture holes 238 are adapted to be aligned with the suture cavity 210 to allow insertion of the sutures 32 into the suture cavity 210.

Differing from that of the first embodiment, the suture accommodating unit 23 of this embodiment is adapted to accommodate at least two sutures 32, and one or more of them can be chosen for use. The suture accommodating unit 23 has at least two suture holes 238 adapted to be separately aligned with the suture cavity 210. Reference can be made to the foregoing embodiments for structural details of the suture accommodating unit 23, and features thereof can be used in combination with those of this embodiment. The description thereof will not be repeated here, for the sake of brevity. In particular, in the suture accommodating unit 23 of this embodiment, an intended one of the suture holes 238 may be selectively aligned with the suture cavity 210. In this way, an operator may select a desired one of the different sutures 32 inserted in the suture holes 238 for a particular surgical application. In practice, the operator can actively turn the suture accommodating unit 23 to align a desired one of the suture holes 238 with the suture cavity 210, allowing the suture 32 to be pulled out. Specifically, a coil 31 of the suture 32 may be accommodated in the conveyor tip 22, and hooked and retained by the hooking unit 131 of the positioner 1.

Referring to Fig. 23, in an exemplary implementation, the suture accommodating unit 23 includes an outer bobbin 2312 and an inner bobbin 2313. The outer bobbin 2312 is rotatably disposed over the inner bobbin 2313 around an axis thereof and can freely rotate relative to the inner bobbin 2313 in one direction. The sutures 32 are wound on the outer bobbin 2312, and the outer bobbin 2312 is configured to rotate relative to the inner bobbin 2313 in one direction to align the individual suture holes 238 separately with the suture cavity 210.

Referring to Fig. 21, the case 232 defines a corresponding window 2320, through which the operator can insert his/her finger into the case 232 to turn the outer bobbin 2312. In addition, the operator can observe the sutures 32 wound on the outer bobbin 2312 through the window 2320 and select a desired one of them.

Optionally, two matching sets of teeth 2314 are defined respectively on an inner circumferential surface of the outer bobbin 2312 and an outer wall surface of the inner bobbin 2313. These teeth 2314 are adapted to effect unidirectional rotatability of the outer bobbin 2312 relative to the inner bobbin 2313. In the exemplary implementation shown in Fig. 23, the teeth 2314 allow the outer bobbin 2312 to rotate relative to the inner bobbin 2313 only clockwise but not counterclockwise. Accordingly, the operator can turn the outer bobbin 2312 only clockwise to select a desired one of the sutures 32.

Additionally, the sutures 32 are wound on the outer bobbin 2312 in the direction opposite to the direction in which the outer bobbin 2312 is allowed to rotate relative to the inner bobbin 2313. For example, in the exemplary implementation shown in Fig. 23, since the direction in which the outer bobbin 2312 is allowed to rotate relative to the inner bobbin 2313 is clockwise, the sutures 32 are counterclockwise wound on the outer bobbin 2312. With this configuration, when a suture 32 is being pulled distally, the outer bobbin 2312 will not consequently rotate relative to the inner bobbin 2313. It will be appreciated that the above-described the configuration of the teeth 2314 is merely a non-limiting example, and those skilled in the art may appropriately configure the teeth 2314 so that the outer bobbin 2312 is allowed to rotate relative to the inner bobbin 2313 only in the opposite direction. Detail description thereof is, however, omitted herein.

In some implementations, the inner bobbin 2313 may be fixed to the case 232. Although fixing the inner bobbin 2313 to the case 232 allowing a desired one of the sutures 32 to be selected, this cannot provide an indication of whether a suture 32 is successfully hooked and retained by the hooking unit 131.

Preferably, in some other implementations, the inner bobbin 2313 is disposed in the case 232 so as to be rotatable about its own axis therein. Additionally, the conveyor 2 may include an indication unit 26. In this case, when the outer bobbin 2312 is rotating under the drive of a suture 32, the inner bobbin 2313 may be driven thereby to rotate in the same direction. This may in turn cause an end of the indication unit 26 where it is attached to the inner bobbin 2313 to displace relative to the case 232, thereby providing an indication.

Optionally, the indication unit 26 may include a torsion spring 261 and an indication mark 262. A first end 2611 of the torsion spring 261 is coupled to the case 232, and a second end 2612 of the torsion spring 261 is coupled to the inner bobbin 2313. The indication mark 262 is attached to the end of the torsion spring 261 coupled to the inner bobbin 2313 (i.e., the second end 2612). The case 232 has an observation area 236 allowing observation of the indication mark 262.

Additionally, the case 232 may have a rotation stop unit 2371, and the inner bobbin 2313 may have a push post 2311 adapted for abutment and connection of the inner bobbin 2313 with the second end 2612 of the torsion spring 261. When the inner bobbin 2313 is rotating about its own axis in a first direction, it may push the second end 2612 of the torsion spring 261 and thereby cause it to displace relative to the case 232. The rotation stop unit 2371 is adapted to come into abutment with the push post 2311 or the torsion spring 261 after the inner bobbin 2313 has rotated a predetermined angle in the first direction, thereby preventing its further rotation in the same direction. The torsion spring 261, the push post 2311, the rotation stop unit 2371 and the indication mark 262 may be configured similarly to those of the third embodiment and, therefore, need not be described in further detail here.

With the above described arrangement, during use, the operator is allowed to select a desired one of the sutures 32 by clockwise turning the outer bobbin 2312. In this process, due to the presence of the teeth 2314 between the outer bobbin 2312 and the inner bobbin 2313, and since the inner bobbin 2313 is restricted by the case 232, the inner bobbin 2313 will not rotate. Once the coil 31 of the suture 32 is hooked and retained by the hooking unit 131, since the suture 32 is wounded in the direction opposite to the direction in which the outer bobbin 2312 can rotate, it will drive the outer bobbin 2312 to rotate counterclockwise and to in turn cause the inner bobbin 2313 to also rotate counterclockwise through the teeth 2314. Moreover, the push post 2311 of the inner bobbin 2313 drives the second end 2612 of the torsion spring 261 and hence the indication mark 262 to move, providing an indication. In an alternative implementation, the observation area 236 may be provided on a circumference of the cylindrical case 232. The indication mark 262 may, for example, includes red and green sections extending circumferentially around the case 232. In an initial configuration, the red section of the indication mark 262 is aligned with the observation area 236. When the suture 32 is successfully hooked and retained, it will cause the green section of the indication mark 262 to move into alignment with the observation area 236, indicating the success in suture retention. Further, after the current suture 32 is completely pulled out, the indication mark 262 will be biased by the resilient torsion spring 261 back to its initial configuration in which the red section is aligned with the observation area 236 until it is again actuated to provide another indication.

It will be appreciated that the conveyor 2 of this embodiment may adopt a design similar to that of the fourth embodiment, which includes a sleeve 27 and a push rod 28 for working together with the sleeve 27 to deliver the selected suture 32. Alternatively, the suture cavity 210 may be instead configured to be radially open across the length of the conveyor shaft 21. That is to say, the conveyor shaft 21 is trough-shaped rather than tubular. This shape can facilitate placement of the selected suture 32 into the suture cavity 210.

In correspondence to the above-discussed conveyor 2, this embodiment also provides a suture passing instrument including the conveyor 2 as defined above and at least two sutures 32 individually wound on the outer bobbin 2312 of the conveyor 2. Correspondingly, the system of this embodiment includes this suture passing instrument and the positioner 1.

Correspondingly, the method of this embodiment further includes, prior to step S4:
Step SC: aligning a desired one of the suture holes 238 in the suture accommodating unit 23 with the suture cavity 210, winding a portion of a suture 32 inserted in the suture hole 238 aligned with the suture cavity 210 into a coil 31, and retaining the coil 31 at the conveyor tip 22.

### Embodiment 6

In a sixth embodiment of the present invention, there are provided a positioner, a conveyor, a tunnel-type wire passing system and an operating method thereof, which are substantially similar to those of the first embodiment. Only differences of this embodiment from the first embodiment will be described below, and any feature it commonly shares with the first embodiment will not be described again.

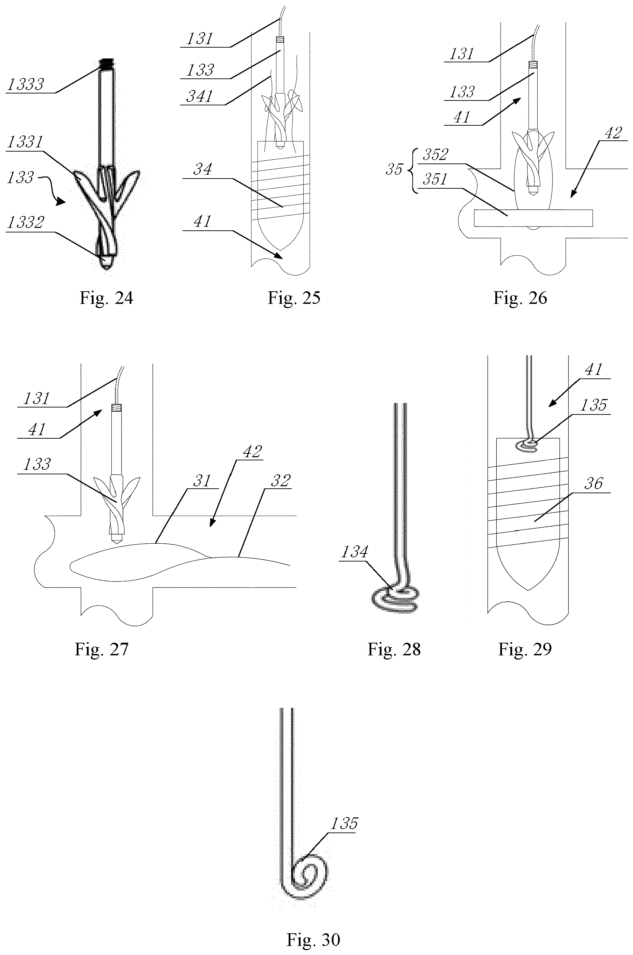

Reference is now made to Figs. 24 to 30. Fig. 24 is a schematic illustration of a double-leaf hook according to the sixth embodiment of the present invention. Fig. 25 is a schematic illustration of the double-leaf hook with a suture anchor caught thereon according to the sixth embodiment of the present invention. Fig. 26 is a schematic illustration of the double-leaf hook with a titanium suture button caught thereon according to the sixth embodiment of the present invention. Fig. 27 is a schematic illustration of the double-leaf hook with a suture hooked and retained thereon according to the sixth embodiment of the present invention. Fig. 28 is a schematic illustration of a flat spiral hook according to the sixth embodiment of the present invention. Fig. 29 is a schematic illustration of the flat spiral hook with an interference screw retained thereon according to the sixth embodiment of the present invention. Fig. 30 is a schematic illustration of a loop hook according to the sixth embodiment of the present invention.

The positioner 1 according to the present sixth embodiment differs in structure from that of the first embodiment. Specifically, the positioner 1 includes a horizontal locating rod 11, a vertical locating rod 12, a guide wire 13, a hooking unit 131 and a drive unit 14. The hooking unit 131 is detachably coupled to a distal end of the guide wire 13. The horizontal locating rod 11, the vertical locating rod 12 and the drive unit 14 are structured and configured similarly to those of the first embodiment and, therefore, need not be described in further detail here. However, the guide wire 13 of the sixth embodiment differs from that of the first embodiment in that the distal hooking unit 131 is a separate component detachably coupled thereto, instead of being integrated therewith.

With this configuration, the hooking unit 131 can be rapidly detached and replaced with another one suitable for a different implant or a suture 32 of a different size, facilitating use in surgical procedures.

As shown in Fig. 24, in an exemplary implementation, the hooking unit 131 includes a double-leaf hook 133 having two extensions 1331 each extending proximally while gradually curving outwardly. The extensions 1331 are adapted to hook and retain a suture 32 or a tensioning suture of an implant. Preferably, the two extensions 1331 extend helically in order to cause less damage to a bone tunnel when rubbing against it.

Fig. 25 shows a suture anchor 34 which is hooked and retained by the double-leaf hook 133 as an example of the implant. As a common-used orthopedic implant, the suture anchor 34 can be delivered into the horizontal tunnel 42 with the conveyor tip 22 of the conveyor 2, where a tensioning suture 341 of the suture anchor 34 can be hooked and retained on the two extensions 1331 of the double-leaf hook 133 of the hooking unit 131.

Fig. 26 shows a titanium suture button 35 which is hooked and retained by the double-leaf hook 133 as another example of the implant. The titanium suture button 35 includes a button body 351 and several tensioning sutures 352 passed through the button body. It can be delivered into the horizontal tunnel 42 with the conveyor tip 22 of the conveyor 2. For example, the button body 351 of the titanium suture button 35 may be held on the retaining fork shown in Fig. 11 and inserted into the horizontal tunnel 42 until it reaches the intersection of the horizontal tunnel 42 and the vertical tunnel 41, where the double-leaf hook 133 can catch and retain the tensioning sutures 352 of the titanium suture button 35 with its two extensions 1331.

It will be appreciated that the double-leaf hook 133 can also be used to directly hook and retain a coil 31 formed of a wound portion of a suture 32, as shown in Fig. 27.

With continued reference to Figs. 24 and 25, the double-leaf hook 133 is preferred to further include a distally-extending adapter portion 1332 for conformal engagement with the implant. Preferably, the adapter portion 1332 is distally tapered and blunt. Referring to Fig. 26, the implant (e.g., the suture anchor 34 or an interference screw) typically defines, at its proximal end, a recess (e.g., a hexagon socket) to be accessed by a catcher. The conformal engagement with the implant may be accomplished by inserting the blunt adapter portion 1332 into the recess of the implant. Here, by "blunt", it is intended to mean that the tapered portion has a blunt tip, which can cause less damage to a bone tunnel when rubbing against it.

Referring to Fig. 28, in an exemplary implementation, the hooking unit 131 includes a flat spiral hook 134 in the shape of a flat spiral defining a plane perpendicular to an axis of the hooking unit 131. The flat spiral hook 134 is adapted to hook and retain a suture 32 or a tensioning suture of an implant. The perpendicularity of the plane defined by the flat spiral hook 134 to the axis of the hooking unit 131 means that it forms an end flange of the hooking unit 131, which can facilitate the hooking and retention of sutures of various sizes and tensioning sutures of various implants. Preferably, the hooking unit 131 is an integral component with greater mechanical strength.

In addition, the guide wire 13 is able to rotate about its own axis relative to the vertical channel 120 and thereby drive the hooking unit 131 to rotate relative to the vertical tunnel 41. Referring to Fig. 29, the flat spiral hook 134 may be turned to screw into an implant made of a soft material, such as an absorbable interference screw 36. In some cases, the guide wire 13 and hence the flat spiral hook 134 coupled to its distal end may be turned to screw the flat spiral hook 134 into an implant such as an absorbable interference screw 36. Of course, the aforementioned double-leaf hook 133 may also be rotated by turning the guide wire 13 so that it is more likely to succeed in catching a suture.

Referring to Fig. 30, in another exemplary implementation, the hooking unit 131 includes a loop hook 135 defining a plane parallel to the axis of the hooking unit 131. The loop hook 135 is adapted to hook and retain a suture 32 or a tensioning suture of an implant. The parallelism of the of the plane defined by the loop hook 135 to the axis of the hooking unit 131 means that it can be made with a slightly smaller outer radial dimension than the aforementioned flat spiral hook 134. This facilitates its operation within a narrower tunnel and makes it more likely to succeed in catching a suture. Preferably, the hooking unit 131 is an integral component with greater mechanical strength.

Preferably, the hooking unit 131 is threadedly coupled to the guide wire 13. Optionally, the hooking unit 131 may define a threaded coupling feature 1333 at its proximal end, which enables the hooking unit 131 to be quickly and easily assembled with or disassembled from a corresponding feature of the guide wire 13. In practice, different hooking unit 131 suitable for different implants or sutures 32 can be quickly interchanged to satisfy various needs.

It is to be noted that the several examples of the hooking unit 131 are presented merely for the purpose of illustration and are not intended to limit the structure of the hooking unit 131 in any sense. The hooking unit 131 may further include other components not described herein. Those skilled in the art may appropriately modify the hooking unit 131, depending on practical circumstances. Similarly, the positioner 1 of this embodiment may be combined with the conveyor 2 of any of the foregoing embodiments to form a tunnel-type wire passing system. However, the description thereof will not be repeated here, for the sake of brevity.

It is to be noted that the foregoing several embodiments may be combined. The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A positioner, comprising a first locating rod, a second locating rod, a guide wire and a drive unit,
the first locating rod defining a first channel extending therethrough along an axial direction of the first locating rod,
the second locating rod connected to the first locating rod, a distal end of the second locating rod protruding beyond a distal end of the first locating rod, the second locating rod defining a second channel extending therethrough along an axial direction of the second locating rod, a distal end of the second channel extending in a direction intersecting a direction in which a distal end of the first channel extends;
the guide wire movably inserted in the second channel along the axial direction of the second locating rod, the guide wire provided at a distal end thereof with a connecting unit,
the drive unit connected to the guide wire, the drive unit adapted to drive the guide wire to move in the axial direction of the second locating rod.

2. The positioner according to claim 1, wherein the direction in which the distal end of the second channel extends forms an angle in the range of 10° to 180° with the direction in which the distal end of the first channel extends.

3. The positioner according to claim 1, wherein the connecting unit comprises a mechanical retainer or a bonding means.

4. The positioner according to claim 1, wherein the mechanical retainer is a hooking unit.

5. The positioner according to claim 1, wherein the first locating rod is connected to the second locating rod and is movable in the axial direction of the first locating rod.

6. The positioner according to claim 1, further comprising a locating rod sleeve defining a sleeve bore extending therethrough along an axial direction of the sleeve bore, an internal contour of the sleeve bore matching in shape with an external contour of the first locating rod, wherein the first locating rod is movably inserted in the sleeve bore along the axial direction of the locating rod sleeve, and the second locating rod is fixedly connected to the locating rod sleeve.

7. The positioner according to claim 1, wherein the second locating rod comprises a curved section and a straight section connected to a proximal end of the curved section, the straight section extending in parallel to the axial direction of the first locating rod.

8. The positioner according to claim 1, wherein the first locating rod has at a distal end thereof an abutment surface configured to be complementary in shape to an object of interest and for abutment with a surface of the object of interest.

9. The positioner according to claim 8, wherein the first locating rod is provided at the distal end thereof with an engagement unit configured to pierce the surface of the object of interest and engage with the object of interest.

10. The positioner according to claim 1, further comprising a handle, which surrounds a proximal end of the first locating rod and extends in a form of a straight-line shape.

11. The positioner according to claim 1, wherein the guide wire is flexible and the connecting unit has rounded, smooth edges.

12. A conveyor, comprising a conveyor shaft and a conveyor tip,
the conveyor tip disposed at a distal end of the conveyor shaft and configured to accommodate an implant or a coil formed from a suture,
the conveyor shaft configured to be movably inserted in the first channel in the positioner according to any of claims 1 to 11, the conveyor tip configured to protrude out of the first locating rod from the distal end thereof to allow the connecting unit of the positioner to catch the coil or the implant accommodated in the conveyor tip.

13. The conveyor according to claim 12, wherein the conveyor shaft defines a suture cavity extending therethrough along an axial direction of the conveyor shaft, and the suture cavity is configured to accommodate part of the suture.

14. The conveyor according to claim 12, comprising a suture accommodating unit, which is connected to a proximal end of the conveyor shaft and configured to accommodate a proximal end portion of the suture.

15. The conveyor according to claim 12, further comprising a stop unit secured to the conveyor shaft, the stop unit configured to come into abutment with a corresponding portion of the positioner and restrict distal displacement of the conveyor shaft relative to the first channel, wherein upon the stop unit coming into abutment with the corresponding portion of the positioner, the conveyor tip is located at an intersection of the direction in which the distal end of the second channel extends and the direction in which the distal end of the first channel extends.

16. The conveyor according to claim 12, wherein the conveyor tip comprises a first recess configured to accommodate the coil and a second recess configured to allow the connecting unit to pass though.

17. The conveyor according to claim 12, wherein the conveyor tip further comprises a retaining fork with a distal end defining a locating retainer and at least two fork prongs which are connected by the locating retainer to form a closed retaining slot for capturing the coil of the suture or accommodating the implant.

18. The conveyor according to claim 17, wherein the conveyor tip comprises two said retaining forks which are disposed in opposition to each other.

19. A tunnel-type wire passing system, comprising the positioner according to any of claims 1 to 11 and the conveyor according to any of claims 12 to 18, wherein the conveyor shaft in the conveyor is configured to be inserted in the first channel of the positioner, and the connecting unit of the positioner is configured to connect the coil or the implant accommodated in the conveyor tip.

20. An operating method of the tunnel-type wire passing system according to claim 19, the operating method comprising:
forming a second tunnel in an object of interest;
inserting the distal end of the second locating rod of the positioner into the second tunnel so that the direction in which the distal end of the second channel extends coincides with an axial direction of the second tunnel;
forming a first tunnel in the object of interest along a direction in which the first channel in the positioner extends so that the first tunnel intersects the second tunnel;
inserting the conveyor shaft of the conveyor into the first channel so that the distal end of the conveyor shaft is located within the first tunnel and that the conveyor tip of the conveyor is located at the intersection of the second tunnel and the first tunnel;
using the drive unit to drive the connecting unit to proximally move and connect the coil or the implant accommodated in the conveyor tip;
proximally pulling and withdrawing the conveyor so that the coil or the implant is placed in the second tunnel; and
proximally pulling the positioner until part of the coil or the implant is pulled out of the second tunnel.

21. The operating method of the tunnel-type wire passing system according to claim 20, further comprising, after the coil is pulled out of the second tunnel,
using the drive unit to drive the connecting unit to distally move; and
detaching the coil or the implant from the connecting unit, and separating the coil or the implant from the positioner.
